# FASCICULE DE BREVET EUROPEEN

(11) **EP 0 200 655 B1**
(45) Date de publication et mention de la délivrance du brevet: **26.04.1995**
(21) Numéro de dépôt: 86400961.8
(22) Date de dépôt: 02.05.1986
(51) Int. Cl.: C12N 15/00, C07H 21/04, C12N 1/16, C12P 21/02, A61K 38/00

(54) **Vecteurs d'expression et de sécrétion de l'hirudine par les levures transformées**
Expressions- und Ausscheidungsvektoren für Hirudin mittels transformierter Hefe
Expression and secretion vectors for hirudine by way of transformed yeasts

(30) Priorité: 02.05.1985 FR 8506672
(43) Date de publication de la demande: 05.11.1986
(73) Titulaire: TRANSGENE S.A., 67082 Strasbourg Cédex (FR)
(72) Inventeur: Loison, Gérard, F-67000 Strasbourg (FR); Tolstoshev, Paul Biotechnology, East Roseville Sydney 2069 (AU); Lemoine, Yves, F-67100 Strasbourg (FR); Lecocq, Jean-Pierre, F-67116 Reichstett (FR)
(74) Mandataire: Keller, Günter, Dr.

(56) Documents cités:
- EP-A- 0 116 201
- EP-A- 0 123 294
- EP-A- 0 123 544
- EP-A- 0 129 073
- CHEMICAL ABSTRACTS, vol. 100, no. 19, 7 mai 1984, page 227, abrégé no. 152937g,Columbus, Ohio, US; J. DODT et al.: "The complete amino acid sequence ofhirudin, a thrombin specific inhibitor. Application of colorcarboxymethylation", & FEBS LETT. 1984, 165(2), 180-4

## Description

La présente invention concerne des vecteurs d'expression de la séquence d'ADN codant pour une hirudine ou des analogues de l'hirudine, la sécrétion de l'hirudine dans le milieu de culture des levures transformées par ces vecteurs et des procédés permettant d'obtenir par fermentation une hirudine active ainsi que l'hirudine obtenue.

L'activité anti-coagulante qui se trouve dans les glandes salivaires des sangsues médicinales, Hirudo medicinalis, provient d'un petit polypeptide que l'on appelle l'hirudine (1). Cet inhibiteur très spécifique et très efficace de la thrombine a été largement étudié ces derniers temps car il présente potentiellement un agent thérapeutique très intéressant. Toutefois, la difficulté extrême et le coût de son isolement et de sa purification ont empêché qu'il soit utilisé plus largement, ou même qu'il puisse être étudié sur le plan clinique.

La possibilité de production de l'hirudine par le clonage des gènes, et leur expression grâce à la technique des ADN recombinants, a déjà été montrée par le clonage d'un gène naturel de sangsue codant pour l'hirudine et l'expression dans le microorganisme E. coli (Demande de brevet français n° 84 04755 au nom de la Demanderesse déposée le 27 mars 1984). Même si un peptide ayant une activité biologique a pu être produit dans E. coli, il est très important de produire de l'hirudine dans d'autres types de microorganismes. En effet, l'utilisation de l'hirudine en clinique exige une très grande pureté du produit, et l'élimination des contaminants pyrogéniques pourraît poser des problèmes à la purification de l'hirudine à partir d'extraits du colibacille.

De plus, l'hirudine synthétisée par E. coli reste intracellulaire et doit donc être purifiée à partir d'un très grand nombre de peptides de E. coli. Pour ces raisons, il était intéressant de faire exprimer le gène de l'hirudine dans la levure qui ne produit pas de substances pyrogènes ou toxiques pour l'homme et qui est capable de sécréter des protéines dans le milieu de culture

Le mécanisme d'action de l'hirudine comme anti-coagulant commence seulement à être compris. Le substrat pour la fixation de l'hirudine est la thrombine, qui est une enzyme protéolytique, qui, par activation (par le facteur X activé) à partir de sa forme zymogène, la prothrombine, coupe le fibrinogène dans le flux circulatoire pour le transformer en fibrine qui est nécessaire à la formation du caillot de sang. La constante de dissociation du complexe thrombine-hirudine 1:1 (0,8 x 10⁻¹⁰) indique une association extrêmement forte entre ces molécules (2). Pratiquement, on peut considérer le complexe non-covalent entre ces deux molécules comme indissociable in vivo.

L'hirudine est un inhibiteur très spécifique de la thrombine, avec une affinité beaucoup plus élevée que le substrat naturel, le fibrinogène. En outre, il n'est pas nécessaire d'avoir d'autres facteurs de coagulation ou d'autres constituants du plasma. L'activité anti-thrombinique spécifique et très importante de l'hirudine rend évidente son application clinique en tant qu'anti-coagulant.

L'hirudine a été étudiée de façon très importante chez l'animal pour ses propriétés anti-coagulantes. L'étude la plus détaillée (3) décrit l'activité de l'hirudine dans la prévention des thromboses veineuses, des occlusions vasculaires et des coagulations intravasculaires disséminées (DIC) chez le rat. L'hirudine est bien tolérée par les rats, les chiens, les lapins et les souris lorsqu'elle est sous forme très purifiée et injectée par voie intra-veineuse. La DL₅₀ dans les souris est supérieure à 500 000 U/kg de poids corporel (c'est-à-dire 60 mg/kg). Une autre étude (4) indique que les souris tolèrent des doses allant jusqu'à 1 g/kg et que les lapins tolèrent à la fois par voie intraveineuse et par voie sous-cutanée jusqu'à 10 mg/kg. Chez les souris, des injections répétées pendant une période de deux semaines ne conduisent pas à des réactions de sensibilisation.

En outre, l'hirudine chez l'animal expérimental est rapidement éliminée (demi-vie de l'ordre de 1 heure) encore sous forme biologiquement active par l'intermédiaire des reins (3).

Deux autres études indépendantes, l'une utilisant les chiens (5) et l'autre (6) démontrant l'activité de l'hirudine dans la prévention des DIC chez les rats, sont en accord avec les résultats positifs de Markwardt et de ses collaborateurs. Ces chercheurs ont récemment publié la première analyse in vivo des effets de l'hirudine naturelle sur le système hémostatique humain (7). Le matériel a montré les effets biologiques attendus sans indication d'effets secondaires toxiques.

On a aussi pu démontrer que l'hirudine prévient les DIC induits par les endotoxines dans les cochons (8) et ainsi constitue une solution potentielle aux problèmes très sérieux posés par les endotoxinémies qui conduisent à une mortalité élevée chez les cochons.

Une seule publication très récente (9) décrit l'administration intraveineuse et sous-cutanée d'hirudine à l'homme. Six volontaires ont été utilisés pour évaluer la pharmacocinétique et les effets sur le système hémostatique d'une dose unique (1 000 AT-U/kg) d'hirudine. L'hirudine administrée par voie intraveineuse présente une demi-vie de 50 minutes et on retrouve 50 % de l'hirudine sous forme active dans l'urine, pendant 24 heures après l'injection. On observe une prolongation du temps de coagulation (mesuré in vitro, pour la thrombine, la thromboplastine et la prothrombine) en fonction de la concentration d'hirudine dans le plasma, ce qui montre que la molécule garde son activité biologique dans la circulation du sujet. On n'observe pas de changement dans le nombre de plaquettes, dans le taux de fibrinogène ni dans le système fibrinolytique. Les injections d'hirudine sous-cutanées comme intraveineuses sont bien tolérées et ne provoquent pas d'effet secondaire. Pour tester l'apparition éventuelle de réactions allergiques, 2 injections intracutanées ont été administrées aux mêmes sujets, à 4 semaines d'intervalle ; on n'a observé aucun signe de sensibilisation. De plus, on ne détecte pas d'anticorps anti-hirudine dans le sérum.

Ces études suggèrent que l'hirudine peut constituer un agent clinique intéressant comme anti-coagulant. La pré-phase de coagulation du sang n'est pas affectée compte tenu de la haute spécificité de l'action de l'hirudine. L'activité anti-thrombinique est dépendante de la dose et l'effet de l'hirudine est rapidement réversible compte tenu de son élimination rénale rapide. On a pu mettre en évidence que l'hirudine est très supérieure à l'héparine pour le traitement des DIC (3, 6) comme cela pouvait être attendu compte tenu du fait que la DIC est accompagnée par une décroissance de l'anti-thrombine III (un co-facteur nécessaire pour l'action de l'héparine) et un relarguage du facteur de plaquette 4 qui est un agent très efficace anti-héparine.

Une étude a mis en évidence la possibilité que l'hirudine puisse être absorbée par la peau des êtres humains (10) bien que les résultats obtenus restent quelque peu difficiles à interpréter.

Des préparations commerciales d'extraits bruts acellulaires de sangsues sont disponibles en pommade (Hirucrème, Société Nicholas, France ; Exhirud-Blutgel, Plantorgan Werke, RFA), mais des tests complémentaires avec des doses plus importantes d'un matériel hautement purifié sont nécessaires pour établir s'il s'agit là d'une voie d'administration intéressante. De façon générale, les voies d'administration préférées sont les voies intraveineuses, intramusculaires et la voie percutanée. D'autres voies d'administration ont été rapportées pour l'hirudine, notamment la voie orale (BSM N° 3 792 M).

Ce produit peut également, en combinaison avec d'autres composants, être utilisé dans le traitement du Psoriasis et d'autres désordres cutanés du même type comme cela est décrit dans le DOS 2 101 393.

L'hirudine peut être, en outre, utilisée à titre d'anti-coagulant dans les tests cliniques en laboratoires et comme outil de recherche. Dans ce cas, la haute spécificité pour une étape unique dans la coagulation du sang peut présenter un avantage considérable par rapport aux anti-coagulants utilisés le plus souvent et dont l'action est beaucoup moins spécifique.

En outre, l'hirudine peut être très utile comme agent anti-coagulant dans les circuits extra-corporels et dans les systèmes de dialyses où elle peut présenter des avantages considérables par rapport aux autres anti-coagulants, en particulier s'il peut être immobilisé sous forme active sur la surface de ces systèmes circulatoires artificiels.

L'activité de fixation de l'hirudine sur la thrombine peut, en outre, permettre la protection indirecte de facteurs de coagulation tels que le facteur VIII, lors de sa purification.

Finalement, l'utilisation de l'hirudine marquée peut constituer une méthode simple et efficace pour mesurer les taux de thrombine et de prothrombine. En particulier, l'hirudine marquée peut être utilisée afin de visualiser les caillots en formation car le phénomène de coagulation implique la transformation de la prothrombine circulante en thrombine au site de formation, l'hirudine marquée venant se fixer sur la thrombine et pouvant être visualisée.

Il est en outre possible de prévoir l'utilisation directe des levures transformées à titre de médicament libérant de l'hirudine, par exemple en étalant une crème contenant lesdites levures qui sécrètent de l'hirudine sur la peau.

En résumé, l'hirudine selon l'invention présente un grand nombre d'applications possibles :
1°) comme anti-coagulant dans des conditions thrombotiques critiques pour la prophylaxie et la prévention de l'extension des thromboses existantes ;
2°) comme anti-coagulant pour réduire les hématomes et les enflures après les micro-chirurgies, situations où l'on fait une utilisation importante de sangsues vivantes ;
3°) comme anti-coagulant dans des systèmes de circulation extracorporelle et comme agent anti-coagulant pour revêtir des biomatériaux synthétiques ;
4°) comme anti-coagulant dans les tests cliniques des échantillons de sang dans les essais de laboratoires ;
5°) comme anti-coagulant dans la recherche clinique sur la coagulation et comme outil expérimental ;
6°) comme agent topique possible pour l'application cutanée dans le traitement des hémorroïdes, des varices et des oedèmes ;
7°) comme composant dans le traitement des Psoriasis et autres désordres apparentés ;
8°) enfin, l'hirudine peut être utilisée pour fixer la thrombine lors de la conservation du sang et de la préparation de dérivés du sang (plaquettes, facteurs VIII et IX).

A titre indicatif, l'hirudine peut être utilisée dans les compositions thérapeutiques à des concentrations correspondant à 100-50 000 U anti-thrombiniques/kg par jour.

L'hirudine étant soluble dans l'eau, il est aisé d'obtenir des compositions pharmaceutiques injectables ou applicables par d'autres voies en utilisant des supports et véhicules pharmaceutiquement acceptables.

Enfin, il est possible d'utiliser de l'hirudine marquée soit par un marquage radioactif ou par tout autre type de marquage enzymatique ou fluorescent réalisé par des techniques connues afin d'effectuer des dosages in vitro ou de l'imagerie in vivo, en particulier pour visualiser la formation de caillots.

Une préparation d'hirudine à partir de l'animal entier a été utilisée pour déterminer la séquence d'acides aminés de la protéine (11,12). Dans les expériences suivantes on a cloné un gène qui s'exprime en ARN messager dans les têtes des sangsues à jeun. Ce gène porte une information pour une protéine (hirudine variante 2 ou HV-2) dont la séquence est significativement différente de celle que l'on trouve dans tout le corps de l'animal (protéine variante appelée HV-1). Il y a 9 différences en résidus aminoacyles entre HV-1 et HV-2 et les différences entre les deux résidus NH₂-terminaux (val-val ou ile-thr) peuvent expliquer les apparentes contradictions dans la littérature concernant l'extrémité NH₂-terminale de l'hirudine (13).

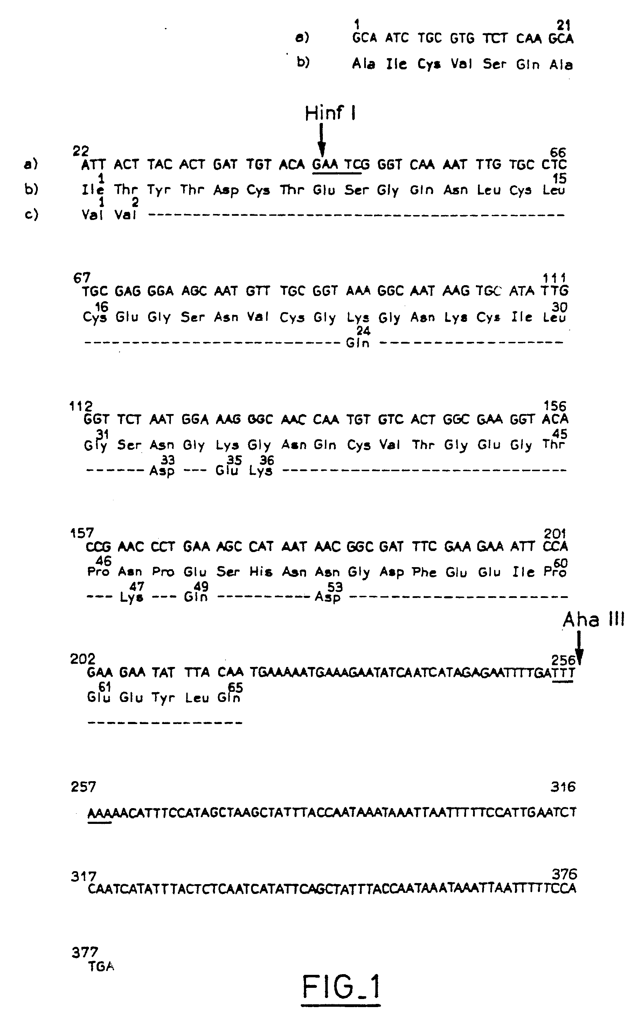

La figure 1 montre les séquences de DNA du plasmide recombinant pTG717 qui contient une copie du cDNA correspondant au mRNA de HV-2, ainsi que la séquence en acides aminés déduite de la séquence du DNA, en b), et les différences entre cette séquence et la séquence en acides aminés de HV-1, en c).

Un variant de l'hirudine HV1 est décrit dans FEBS LETTERS, 1984, vol. 105 (2), pages 180-184.

Il convient de noter que la séquence du cDNA n'est probablement pas complète et qu'il peut exister une séquence-signal en amont du début de la protéine mature.

L'expression du cDNA d'HV-2 dans les microorganismes montre que la protéine correspondante a une activité antithrombine.

Bien que les expériences suivantes aient été réalisées avec le variant HV-2, dans ce qui suit on désignera par "hirudine" et "gène codant pour l'hirudine" aussi bien l'une que l'autre des variantes, c'est-à-dire HV-1 ou HV-2 de même éventuellement que d'autres variantes, et les séquences correspondantes, sauf indication contraire.

L'un des objets de la présente invention est la préparation d'hirudine par des levures.

Les levures sont des organismes eucaryotes unicellulaires. Le genre de levure Saccharomyces comprend des souches dont la biochimie et la génétique sont étudiées intensivement en laboratoire ; il comprend également des souches utilisées dans l'industrie alimentaire (pain, boissons alcoolisées, etc.) et produites par conséquent en très grande quantité.

La facilité avec laquelle on peut manipuler la génétique des cellules de Saccharomyces cerevisiae, soit par les techniques classiques, soit par les techniques dérivées du génie génétique, et encore mieux par la combinaison de ces deux types de technique, et la longue histoire industrielle de cette espèce en font un hôte de choix pour la production de polypeptides étrangers.

EP-A-123 294 décrit des vecteurs de transformation de levure assurant la sécrétion de polypeptides précurseurs hybrides permettant la récupération du polypeptide désire.

EP-A-116 201 décrit des vecteurs de sécrétion dans les levures utilisant la séquence leader du facteur alpha.

EP-A-129 073 décrit l'expression par des levures du GRF en utilisant notamment des éléments issus du gène du facteur alpha.

EP-A-123 544 décrit la sécrétion de protéines en utilisant des éléments provenant de gènes du facteur alpha.

C'est pourquoi la présente invention concerne plus particulièrement un bloc fonctionnel d'ADN permettant la préparation de l'hirudine à partir de levure, caractérisé en ce qu'il comporte au moins :
. le gène de l'hirudine ou d'un de ses variants (ci-après gène H) ;
. une séquence d'ADN (Sₜᵣ) comportant les signaux assurant la transcription du gène H par la levure.

Ce bloc fonctionnel, intégré dans un plasmide ou dans les chromosomes d'une levure, de préférence du genre Saccharomyces, pourra permettre, après transformation de ladite levure, l'expression de l'hirudine, soit sous forme active, soit sous forme d'un précurseur inactif, pouvant régénérer l'hirudine par activation.

L'intérêt de Saccharomyces cerevisiae est que cette levure est capable de sécréter quelques protéines dans le milieu de culture, l'étude des mécanismes responsables de cette sécrétion est en plein essor,et on a montré qu'il était possible de faire sécréter à la levure, après manipulations adhoc, des hormones humaines correctement processées et en tous points semblables à celles que l'on trouve dans le sérum humain (14, 15).

Dans le cadre de la présente invention, cette propriété est mise à profit pour obtenir la sécrétion de l'hirudine car ceci présente de nombreux avantages.

Tout d'abord, la levure sécrète peu de protéines, ce qui a l'avantage de permettre, si on arrive à diriger la sécrétion d'une protéine étrangère donnée à un haut niveau, d'obtenir dans le milieu de culture un produit pouvant représenter un pourcentage élevé de protéines totales sécrétées, et donc de faciliter le travail de purification de la protéine recherchée.

Il existe plusieurs protéines ou polypeptides sécrétés par la levure. Dans tous les cas connus, ces protéines sont synthétisées sous forme d'un précurseur plus long dont la séquence NH₂-terminale est décisive pour l'entrée dans le chemin métabolique conduisant à la sécrétion.

La synthèse dans la levure de protéines hybrides contenant la séquence NH₂-terminale d'un de ces précurseurs suivie de la séquence de la protéine étrangère peut, dans certains cas, aboutir à la sécrétion de cette protéine étrangère. Le fait que cette protéine étrangère soit synthétisée sous forme d'un précurseur, donc en général inactif, permet de protéger la cellule contre les effets toxiques éventuels de la molécule recherchée, le clivage qui libère la protéine active ne se faisant que dans des vésicules issues de l'appareil de Golgi qui isolent la protéine du cytoplasme.

L'utilisation des chemins métaboliques conduisant à la sécrétion pour faire produire à la levure une protéine étrangère a donc plusieurs avantages :
1°) il permet de récupérer dans le surnageant de culture un produit raisonnablement pur ;
2°) il permet de protéger la cellule contre les effets toxiques possibles de la protéine mature.
3°) D'autre part, les protéines sécrétées peuvent, dans certains cas, subir des modifications (glycosylation, sulfatation, etc.).

C'est pourquoi les blocs d'expression selon l'invention auront plus particulièrement la structure suivante :
―Sₜᵣ―Lₑₓ―S_{cl}―gène H―
Lₑₓ code pour une séquence leader nécessaire à
l'excrétion de la protéine correspondant au gêne H ;
S_{cl} est une séquence d'ADN codant pour un site de clivage ; en outre, l'élément S_{cl} -gène H peut être répété plusieurs fois.

On a choisi comme exemple de système de sécrétion celui de la phéromone alpha, c'est-à-dire que,dans la séquence précédente, la séquence Lₑₓ provient du gène de la phéromone sexuelle alpha de levure, mais d'autres systèmes pourraient être utilisés (par exemple le système de la protéine Killer) (14).

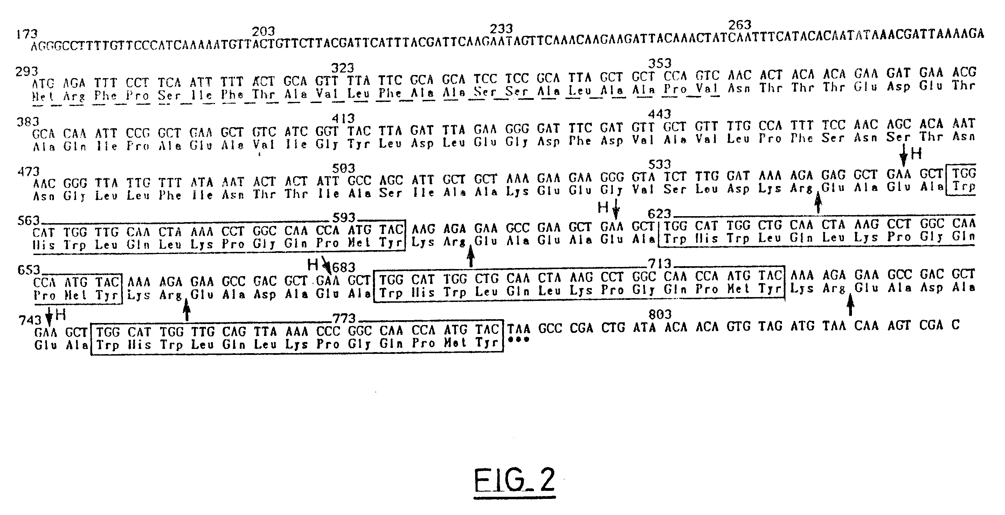

La phéromone sexuelle α de levure est un peptide de 13 amino-acides (encadrés sur la figure 2) qui est sécrété dans le milieu de culture par les levures S. cerevisiae de type sexuel Matα. Le facteur α arrête les cellules de type sexuel opposé(Mata) en phase G1 et induit des changements biochimiques et morphologiques nécessaires à la copulation des deux types de cellules. Kurjan et Herskowitz (17) ont cloné le gène structural du facteur α et ont déduit de la séquence de ce gène que ce facteur α de 13 amino-acides était synthétisé sous forme d'une pré-pro-protéine précurseur de 165 amino-acides (figure 2). Le précurseur contient une séquence hydrophobe amino-terminale de 22 résidus (soulignée en pointillé) suivis d'une séquence de 61 amino-acides contenant 3 sites de glycosylation suivis enfin de 4 copies du facteur α. Les 4 copies sont séparées par des séquences "spacer" et la protéine mature est libérée à partir du précurseur grâce aux activités enzymatiques suivantes :
1°) une endopeptidase de type cathepsin B qui coupe en COOH des dipeptides Lys-Arg (site de coupure indiqué par une flèche grasse) ;
2°) une exopeptidase de type carboxypeptidase B qui coupe les résidus basiques en COOH des peptides excisés ;
3°) une dipeptidyl-aminopeptidase (dite A) qui enlève les résidus Glu-Ala et Asp-Ala.

La séquence nucléotidique de ce précurseur comporte, en outre, 4 sites de restriction HindIII indiqués par une flèche H.

On a réalisé plusieurs fusions entre le gène de la phéromone α et la séquence mature de l'hirudine. Les cellules de levure de type Matα peuvent exprimer ces gènes fusionnés. Les protéines hybrides correspondantes peuvent alors être processées grâce aux signaux qu'elles contiennent et qui proviennent des séquences pré-pro du précurseur de la phéromone α. On s'attend, par conséquent, à récupérer dans le surnageant de culture des polypeptides ayant la séquence de l'hirudine.

Dans une des constructions, la séquence S_{cl} comporte à l'extrémité 3' un codon ATG précédant le gène H ; la protéine fusionnée contient donc une méthionine immédiatement en amont du premier amino-acide de la séquence mature de l'hirudine. Ce polypeptide, après coupure au bromure de cyanogène, génère une molécule d'hirudine que l'on peut rendre active après une étape de renaturation.

Dans d'autres constructions, les signaux de coupure normalement utilisés pour produire la phéromone α servent à produire dans le surnageant de culture des polypeptides possédant une activité anti-thrombine. Ceci est le cas lorsque la séquence S_{cl} comporte à son extrémité 3' deux codons codant pour Lys-Arg, c'est -à-dire AAA ou AAG avec AGA ou AGG ; le polypeptide est coupé par une endopeptidase qui coupe en COOH les dipeptides Lys-Arg, libérant ainsi l'hirudine.

En particulier, l'invention concerne des constructions dans lesquelles la séquence prédédant le gène de l'hirudine code pour l'une des séquences d'amino-acide suivants :
1) Lys Arg Glu Ala Glu Ala Trp Leu Gln Val Asp Gly Ser Met Hirudine ...,
2) Lys Arg Glu Ala Glu Ala Hirudine ...,
3) Lys Arg Glu Ala Glu Ala Lys Arg Hirudine ...
4) Lys Arg Glu Ala Glu Ser Leu Asp Tyr Lys Arg Hirudine... ou
5) Lys Arg Hirudine...

Il est bien entendu possible de prévoir d'utiliser d'autres séquences qui, au niveau des acides aminés, sont coupées sélectivement par une enzyme, sous réserve que ce site de clivage ne soit pas également présent dans l'hirudine elle-même.

Enfin, les blocs d'expression pourront présenter, après le gène H, une séquence de terminateur de levure, par exemple celui du gène PGK.

De façon générale, les blocs d'expression selon l'invention pourront être intégrés dans une levure, en particulier Saccharomyces, soit dans un plasmide à réplication autonome, soit dans le chromosome de la levure.

Lorsque le plasmide est autonome, il comportera des éléments assurant sa réplication, c'est-à-dire une origine de réplication telle que celle du plasmide 2». En outre, le plasmide pourra comporter des éléments de sélection tels que le gène URA3 ou LEU2 qui assurent la complémentation de levures ura3⁻ ou leu2⁻. Ces plasmides peuvent également comporter des éléments assurant leur réplication dans les bactéries, lorsque le plasmide doit être un plasmide "navette", par exemple une origine de réplication telle que celle de pBR322, un gène marqueur tel que Amp^{r} et/ou d'autres éléments connus de l'homme de métier.

La présente invention concerne également des souches de levures transformées par un bloc d'expression selon l'invention, soit porté par un plasmide, soit intégré dans ses chromosomes. Parmi ces levures, il faut citer plus particulièrement les levures du genre Saccharomyces, notamment S. cerevisiae.

Lorsque le promoteur est celui du gène de la phéromone α, la levure sera de préférence de type sexuel Matα. On utilisera, par exemple, une souche de génotype ura3⁻ ou leu2⁻ ou autre, complémenté par le plasmide pour assurer le maintien du plasmide dans la levure par une pression de sélection appropriée.

Bien qu'il soit possible de préparer l'hirudine par fermentation des souches transformées précédentes sur un milieu de culture adapté, par accumulation d'hirudine dans les cellules, il est néanmoins préféré, comme cela ressort de la description précédente, de faire sécréter l'hirudine dans le milieu, soit sous forme mature, soit sous forme d'un précurseur qui devra être processé in vitro.

Cette maturation peut se faire en plusieurs étapes. Tout d'abord, il peut être nécessaire de cliver certains éléments provenant de la traduction de la séquence Lₑₓ, ce clivage sera effectué au niveau de la séquence correspondant à S_{cl}. Comme cela a été indiqué précédemment, l'hirudine mature peut être précédée d'une méthionine qui sera coupée sélectivement par le bromure de cyanogène. Ce procédé est utilisable parce que la séquence codante de l'hirudine ne comprend pas de méthionine.

Il est également possible de prévoir à l'extrémité N le dipeptide Lys-Arg qui est coupé en COOH par une endopeptidase spécifique ; cette enzyme étant active dans le processus de sécrétion, on peut donc obtenir ainsi directement la protéine mature dans le milieu. Mais, dans certains cas, il peut être nécessaire de prévoir un clivage enzymatique après sécrétion en ajoutant une enzyme spécifique.

Dans certains cas, en particulier après un traitement au bromure de cyanogène, il peut être nécessaire de renaturer la protéine en recréant les ponts disulfures. Pour ce faire, le peptide est dénaturé, par exemple au chlorhydrate de guanidinium, puis renaturé en présence de glutathion réduit et oxydé.

Enfin , l'invention concerne l'hirudine obtenue par les procédés selon l'invention.

D'autres caractéristiques et avantages de la présente invention apparaîtront à la lecture des exemples ci-après.

Sur les figures annexées :
- la figure 1: représente la séquence nucléotidique du fragment du cADN de l'hirudine clonée dans pTG 717 ;
- la figure 2: représente la séquence nucléotidique du précurseur de la phéromone sexuelle α ;
- la figure 3: schématise la construction de pTG834 ;
- la figure 4: schématise la construction de pTG880 ;
- la figure 5: schématise la construction de pTG882 ;
- la figure 6: schématise la construction de M13TG882 ;
- la figure 7: schématise la construction de pTG874 ;
- la figure 8: schématise la construction de pTG876 ;
- la figure 9: schématise la construction de pTG881 ;
- la figure 10: schématise la construction de pTG886 ;
- la figure 11: schématise la construction de pTG897 ;
- la figure 12: représente une électrophorèse sur gel d'acrylamide des protéines de PM > 1 000 obtenues dans le milieu après culture de levures transformées par pTG886 et pTG897 ;
- la figure 13: représente une électrophorèse sur gel d'acrylamide des protéines de PM > 1 000 obtenues dans le milieu après culture de levures transformées par pTG886 et pTG897 ;
- la figure 14: schématise la construction de pTG1805 ;
- la figure 15: représente une électroplhorèse sur gel d'acrylamide des protéines de PM > 1000 obtenues dans le milieu après culture de levures transformées par pTG847 et pTG1805 ;

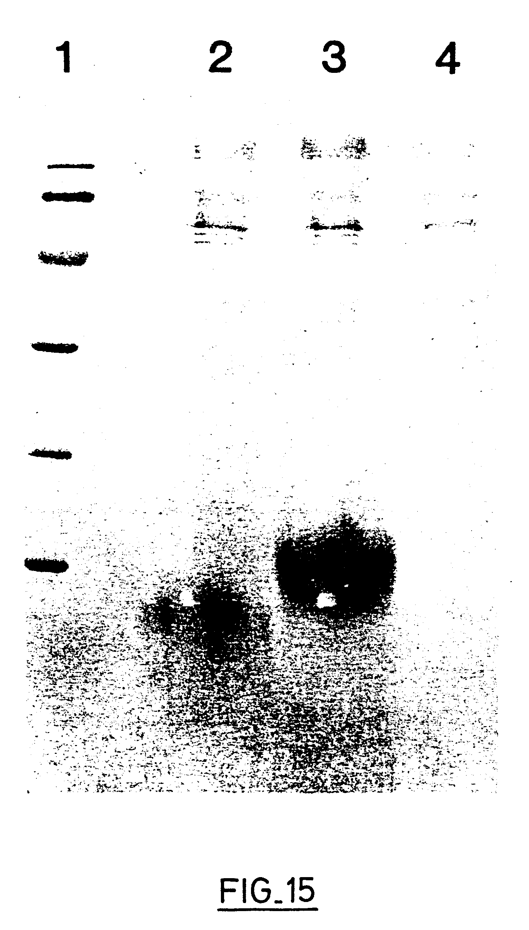

- la figure 15: est un schéma comparant les séquences en aminoacides en aval du premier site de clivage (Lys-Arg) dans différentes constructions.

Les séquences d'amino-acides et de nucléotides ne sont pas reprises dans la présente description pour ne pas l'alourdir mais en font explicitement partie.

### Exemple 1 - Construction de pTG822

Un fragment du cADN de l'hirudine HV-2 a été extrait d'un gel après digestion du plasmide pTG717 avec PstI puis digéré à la fois par les enzymes HinfI et AhaIII. L'enzyme HinfI coupe en aval du premier codon de la séquence mature de l'hirudine HV-2. L'enzyme AhaIII coupe environ 30 paires de basesderrière (3') le codon stop de la séquence de l'hirudine. Le fragment HinfI-AhaIII ainsi obtenu a été isolé sur gel d'agarose puis élué de ce gel.

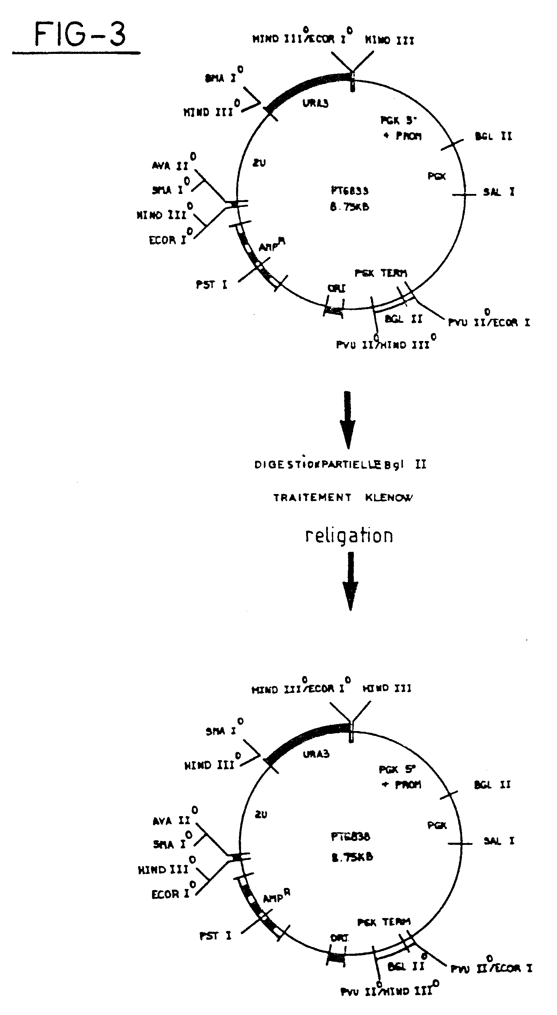

La séquence codant pour la protéine nature a été introduite dans le vecteur pTG880. Le vecteur pTG880 est un dérivé du vecteur pTG838 (figure 3). Le plasmide pTG838 est identique à pTG833 sauf en ce qui concerne le site BglII situé près du terminateur de transcription du PGK. Ce site a été supprimé par l'action de remplissage de la polymérase Klenow pour donner pTG838.

Le plasmide pTG833 est un plasmide navette levure-E. coli. Ce plasmide a été conçu pour exprimer des gènes étrangers dans la levure. Les éléments de base de ce vecteur (figure 3) sont les suivants : le gène URA3 comme marqueur de sélection dans la levure, l'origine de réplication du plasmide 2» de levure, le gène de résistance à l'ampicilline et l'origine de réplication du plasmide pBR322 d'E. coli (ces deux derniers éléments permettant la propagation et la sélection de ce plasmide dans le colibacille), la région flanquante en 5' du gène PGK de levure avec la séquence codante de ce gène jusqu'au site SalI, le fragment SalI-PvuII de pBR322 et le terminateur du gène PGK (20). Ce plasmide a déjà été décrit dans la demande de brevet français n° 84 07125 au nom de la Demanderesse déposée le 9 mai 1984.

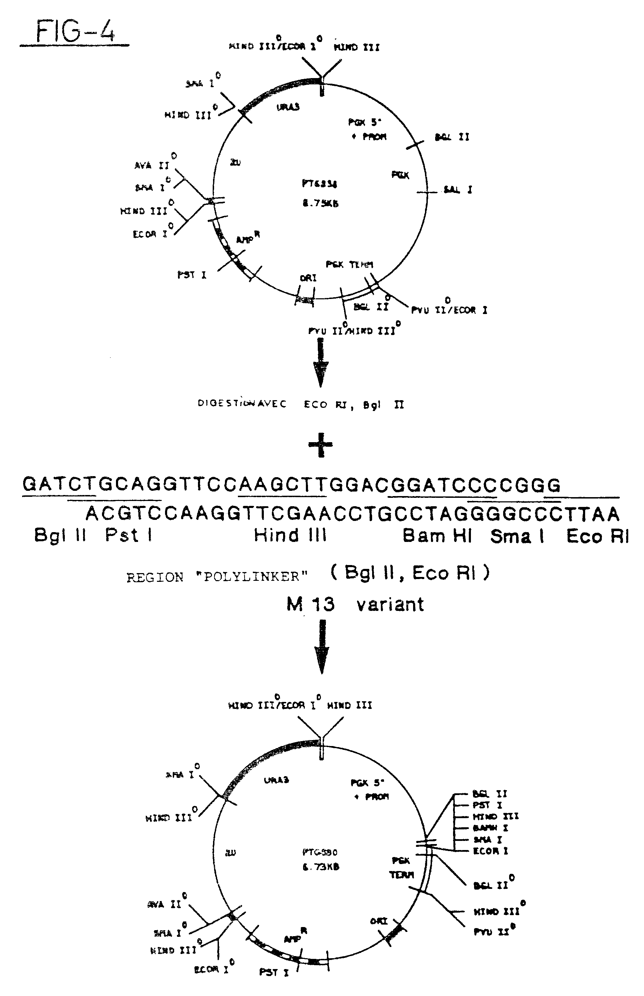

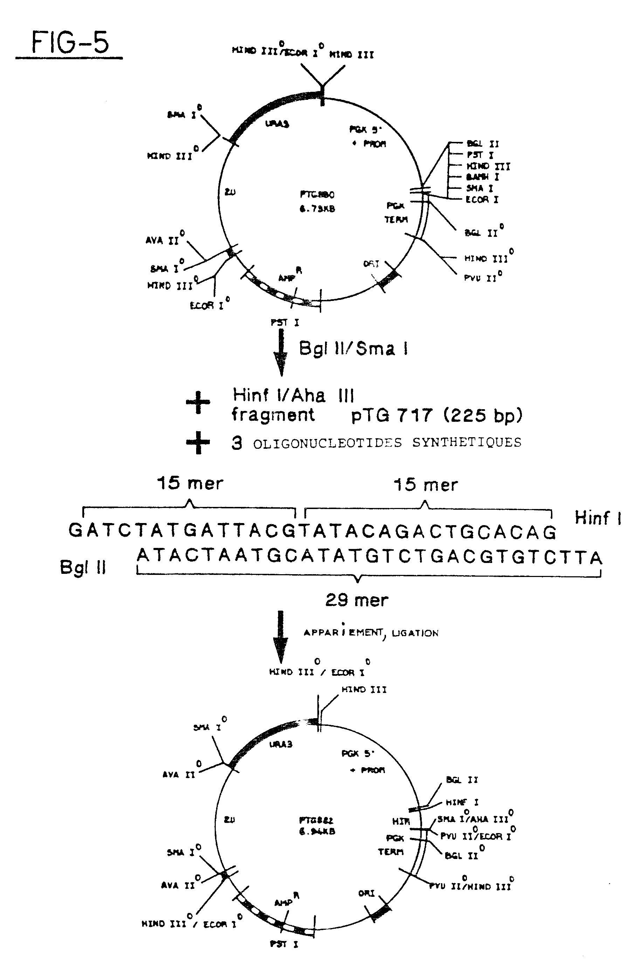

Le plasmide pTG880 a été construit à partir de pTG838 (figure 4) par insertion d'une courte région polylinker (venant d'un bactériophage M13) dans pTG838 coupé par EcoRI et BglII, ce qui permet de placer une série de sites de clonage, dans l'ordre : BglII, PstI, HindIII, BamHI, SmaI et EcoRI, immédiatement après la région 5' flanquant le gène PGK de levure. L'ADN du plasmide pTG880 a été digéré avec BglII et SmaI, et le grand fragment issu de cette digestion a été isolé et élué d'un gel. Le fragment HinfI/AhaIII de pTG717, qui contient la majeure partie de la séquence codante de l'hirudine HV-2 a été mélangé avec du pTG880 digéré en même temps que 3 oligonucléotides synthétiques (figure 5) destinés à reconstituer la région NH₂-terminale de la séquence de l'hirudine, et qui relient le site BglII du vecteur au site HinfI du fragment contenant l'hirudine. Ce mélange a été soumis à un traitement de ligation puis a servi à transformer des cellules de E. coli. On a récupéré le plasmide pTG882 dans des transformants.

Ce plasmide a servi à transformer des cellules de levure dans le but de produire de l'hirudine sous le contrôle du promoteur PGK. Cependant, on n'a décelé aucune activité hirudine dans les extraits bruts des cellules transformées avec ce vecteur. La raison de l'absence de production d'hirudine active n'est pas encore claire. Toutefois, la construction pTG882 a servi comme source de séquence codante d'hirudine pour les vecteurs de sécrétion de levure décrits ci-dessous.

### Exemple 2 - Construction de pTG886 et pTG897

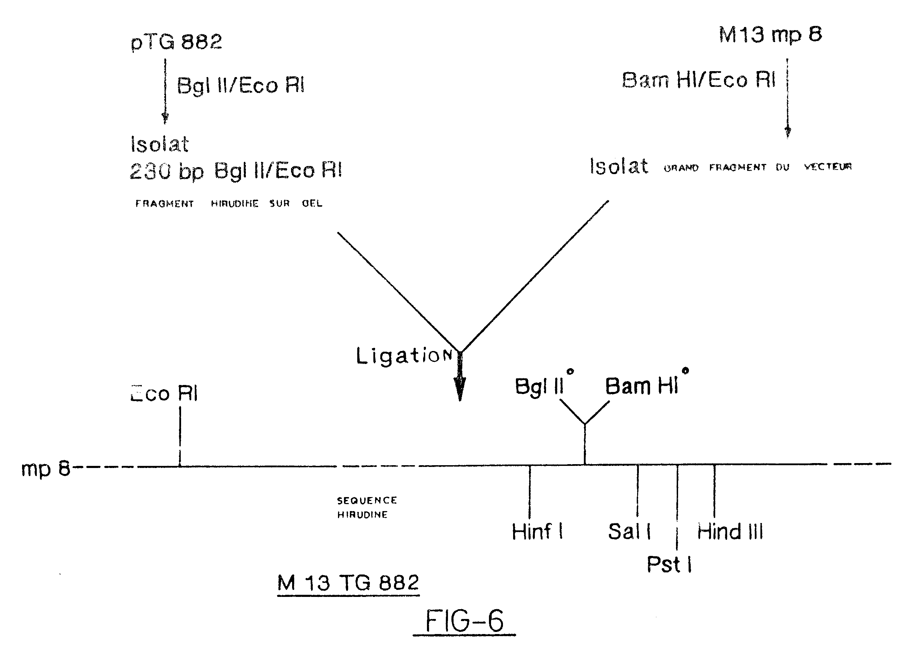

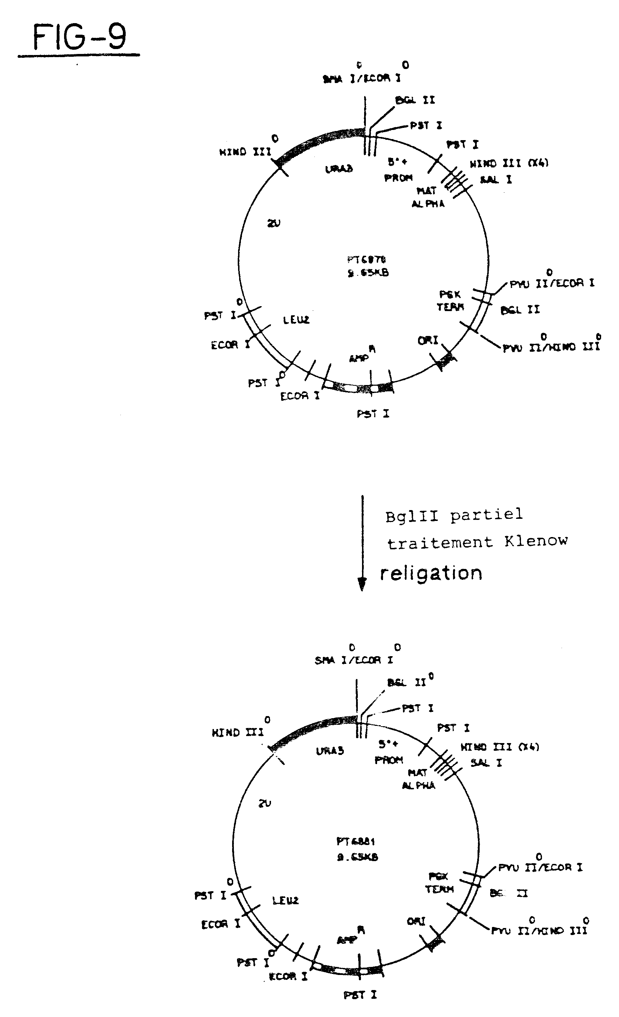

Tout d'abord, le fragment BglII-EcoRI (230 bp) de pTG882 contenant la séquence de l'hirudine a été transféré dans le bactériophage M13mp8 (figure 6) entre les sites BamHI et EcoRI. Ceci a donné le phage M13TG882 à partir duquel un fragment EcoRI-HindIII (environ 245 bp) a pu être isolé. Ce fragment contient l'intégralité de la séquence codante de l'hirudine HV-2, le site de fusion BamHI/BglII et des extrémités cohésives (HindIII-EcoRI) permettant le clonage dans le vecteur de sécrétion de levure pTG881 (figure 9).

Le plasmide pTG881 (10 kb) est un plasmide navette E. coli/levure, se répliquant de manière autonome à la fois dans E. coli et dans les souches Saccharomyces cerevisiae, uvarum et carlbergensis.

L'introduction de ce plasmide dans E. coli permet d'obtenir la résistance à l'ampicilline (et autres antibiotiques de type β-lactame). De plus, ce plasmide porte les gènes LEU2 et URA3 de levure qui sont exprimés dans les souches de E. coli et de Saccharomyces. Donc, la présence de ce plasmide dans E. coli ou dans Saccharomyces permet d'obtenir la complémentation de souches déficientes en β-isopropylmalate déshydrogénase ou en OMP décarboxylase.

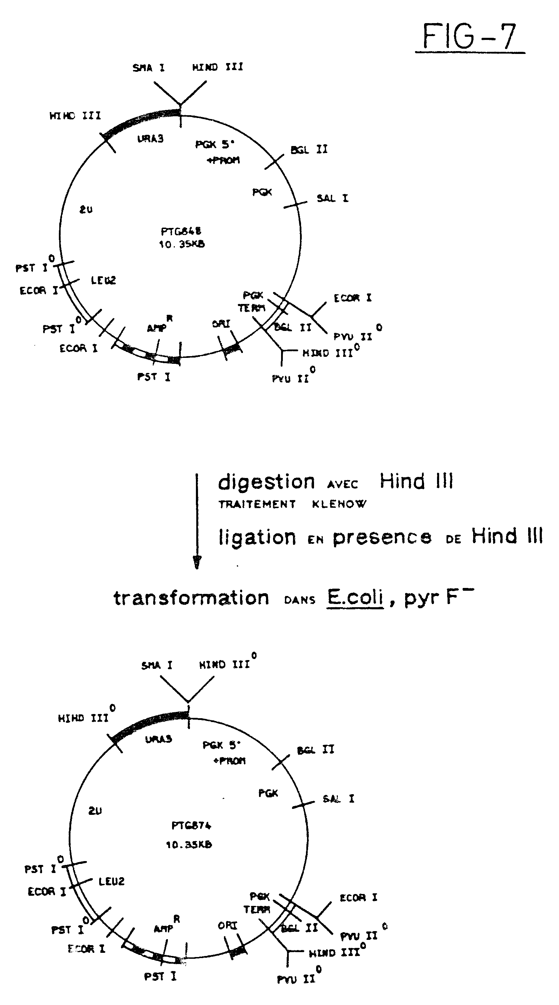

Le plasmide pTG881 est construit de la manière suivante :
Le plasmide de départ est le pTG848 (identique au pTG849 décrit dans le brevet français n° 83 15716 à l'exception du gène ura3 dont l'orientation est inversée), il est constitué des fragments d'ADN suivants (figure 7) :
1°) Le fragment EcoRI-HindIII d'environ 3,3 kb, en provenance du plasmide pJDB207 (18). Le site HindIII correspond à la coordonnée 105 du plasmide 2» forme B, le site EcoRI à la coordonnée 2243. Dans ce fragment se trouve le gène LEU2 inséré par extension polydésoxyadénilate/polydésoxythymidilate dans le site PstI du fragment du 2» forme B (18).
2°) Le fragment HindIII du gène URA3 (19).
3°) Le grand fragment EcoRI (coordonnée 0)-SalI (coordonnée 650) de pBR322. Dans le site PvuII de ce fragment a été inséré le fragment EcoRI-HindIII (dont les extrémités ont été préalablement rendues franches par action de la Klenow en présence des 4 nucléotides) de 510 paires de bases et correspondant à la fin du gène PGK (20). L'extrémité EcoRI arasée du gène PGK lorsque jointe à l'extrémité PvuII de pBR322 régénère un site EcoRI.
4°) Le fragment HindIII-SalI (2,15 kb) du gène PGK (20).

Le plasmide pTG848 a été coupé par HindIII, les extrémités des deux fragments ainsi libérés sont rendues franches par traitement à la Klenow en présence des 4 désoxyribonucléotides. Les deux fragments sont mis en ligation et, avant transformation, ce mélange de ligation est soumis à l'action de HindIII, ce qui permet d'éliminer toute forme de plasmide ayant conservé un (ou deux) site HindIII. On transforme la souche E. coli BJ5183 (pyrF) et on sélectionne les transformés pour la résistance à l'ampicilline et pour le caractère pyr⁺. On obtient ainsi le plasmide pTG874 (figure 7) où les deux sites HindIII sont supprimés, l'orientation du gène URA3 donnant lieu à une transcription dans la même orientation que celle du gène de la phosphoglycérate kinase (PGK).

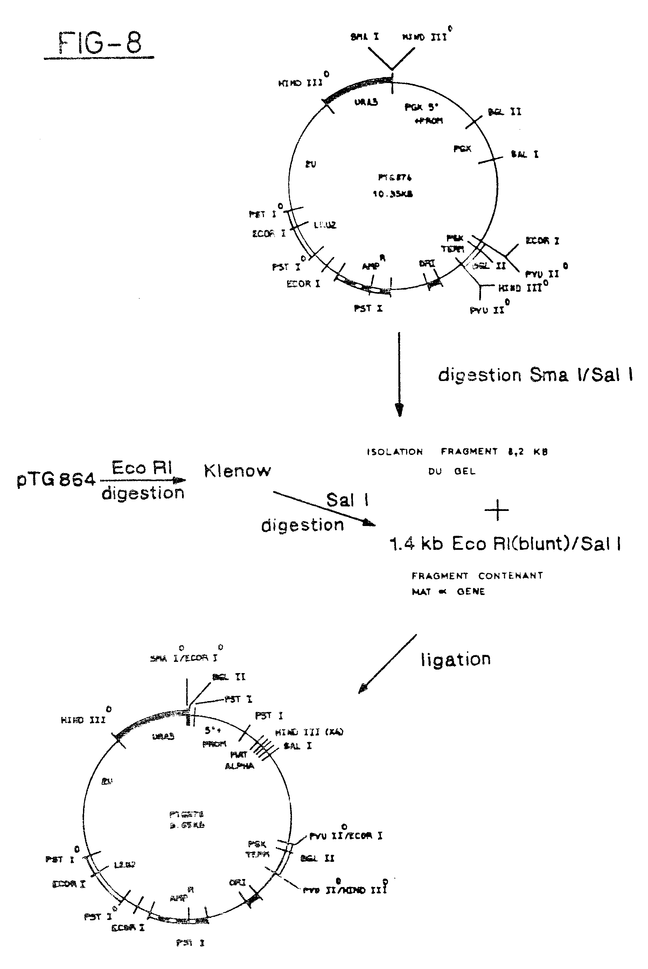

Le plasmide pTG874 est coupé par SmaI et SalI, le fragment de 8,6 kb est ensuite isolé à partir d'un gel d'agarose. Le plasmide pTG864, qui comprend le fragment EcoRI-SalI (environ 1,4 kb) du gène MFα1 cloné dans les mêmes sites de pBR322, est coupé par EcoRI. Les extrémités du plasmide ainsi linéarisé sont rendues franches par action de la Klenow en présence des 4 nucléotides. Une digestion par l'enzyme SalI est ensuite effectuée et le fragment EcoRI (extrémité franche)-SalI correspondant au gène MFα1 est isolé. Ce dernier fragment est mis en ligation avec le morceau SmaI-SalI (8,6 kb) du pTG874 pour donner ainsi le plasmide pTG876 (figure 8).

Afin de supprimer le site BglII proximal de la séquence du promoteur MFα1, une digestion partielle par BglII du plasmide pTG876 a été suivie par l'action de la Klenow en présence des 4 désoxyribonucléotides. Le nouveau plasmide obtenu, pTG881 (figure 9), permet l'insertion de séquences codantes étrangères entre le premier site HindIII du gène MFα1 et le site BglII de la fin du gène PGK.

Lorsque la fusion au site HindIII de l'ADN codant étranger permet d'obtenir une traduction dans la même phase de lecture, la protéine hybride obtenue comprend les parties pré-pro de la phéromone α.

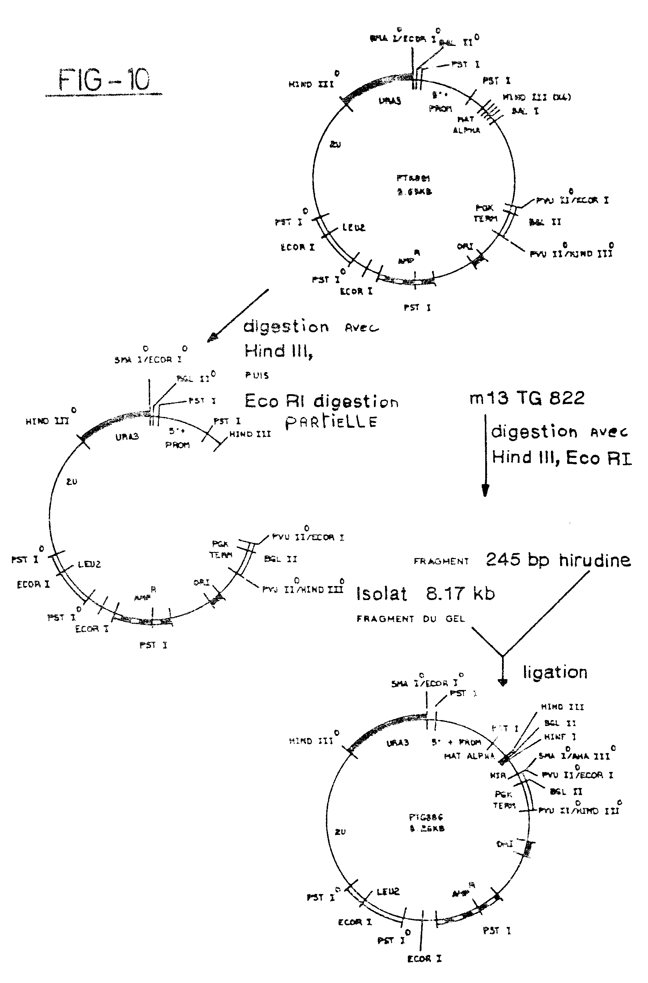

Le clonage dans pTG881 du fragment HindIII-EcoRI qui porte le gène de l'hirudine conduit au plasmide pTG886 (figure 10). Une fois le clonage réalisé, on se trouve avec un site BglII en aval du fragment, ce qui permet de ressortir le fragment sous forme HinfI-BglII, le site HinfI étant le même que celui utilisé plus haut. BglII étant unique dans pTG881, il est très facile de reconstituer l'extrémité 5' de la séquence codante de l'hirudine en utilisant 3 oligonucléotides reconstituant la séquence 5' de l'hirudine et permettant la lecture en phase de la séquence pré-pro de la phéromone α suivie de la séquence mature de l'hirudine.

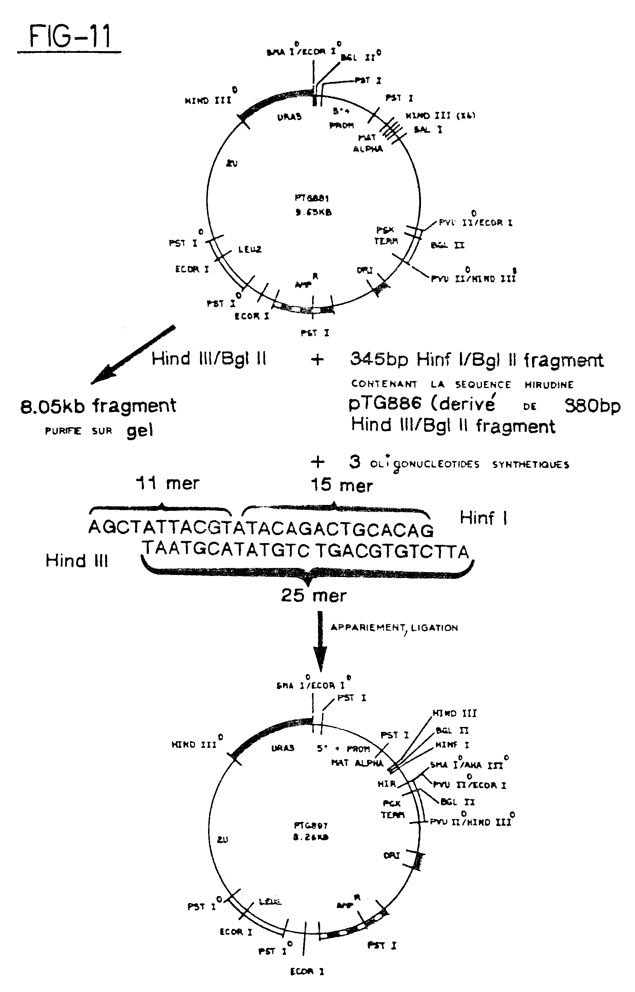

Ce nouveau plasmide est appelé pTG897 (figure 11).

### Exemple 3 - Expression de l'hirudine par les levures

L'ADN de pTG897 a servi à transformer une levure TGY1sp4 (Matα, ura3 - 251 - 373 - 328, his3 - 11-15) en ura⁺ selon une technique déjà décrite (21).

Une colonie transformée a été repiquée et a servi à l'ensemencement de 10 ml de milieu minimum plus casaminoacides (0,5 %). Après 20 heures de culture, les cellules ont été centrifugées, le surnageant dialysé contre de l'eau distillée et concentré par évaporation (centrifugation sous vide).

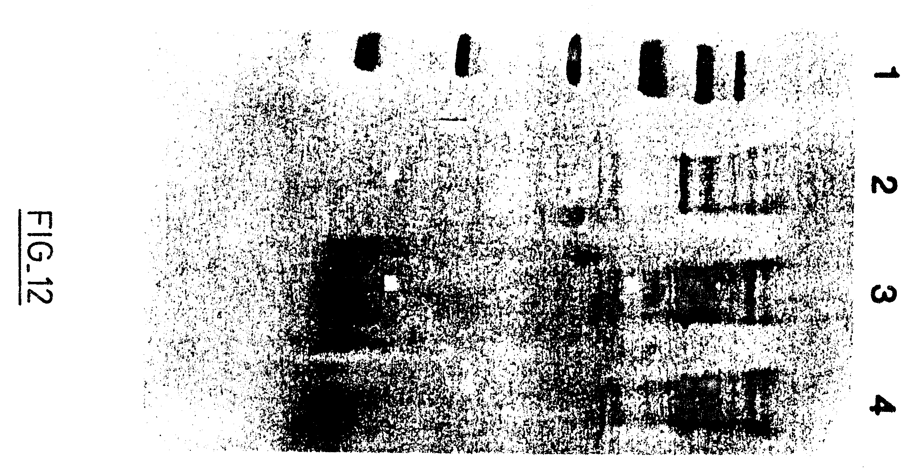

En parallèle, on a traité de la même façon une culture de TGY1sp4 transformée par pTG886 et une culture de TGY1sp4 transformée par un plasmide ne portant pas de séquence pour l'hirudine (TGY1sp4 pTG856). Les culots secs ont été repris dans 50 »l d'eau et 20 »l ont été bouillis en présence de SDS 2,8 % et mercaptoéthanol 100 mM puis déposés sur un gel d'acrylamide-SDS (15 % acrylamide; 0,1 % SDS) (22). Après fixation et coloration au bleu de Coosmassie, on peut déceler des polypeptides qui s'accumulent dans les surnageants des cultures de TGY1sp4/pTG886 et TGY1sp4/pTG897 et qui sont absents dans le surnageant de la culture contrôle (figure 12). De plus, ces séries de peptides supplémentaires se marquent fortement à la cystéine ³⁵S, comme on peut s'y attendre pour des peptides d'hirudine, cette molécule étant très riche en cystéine (figure 10).

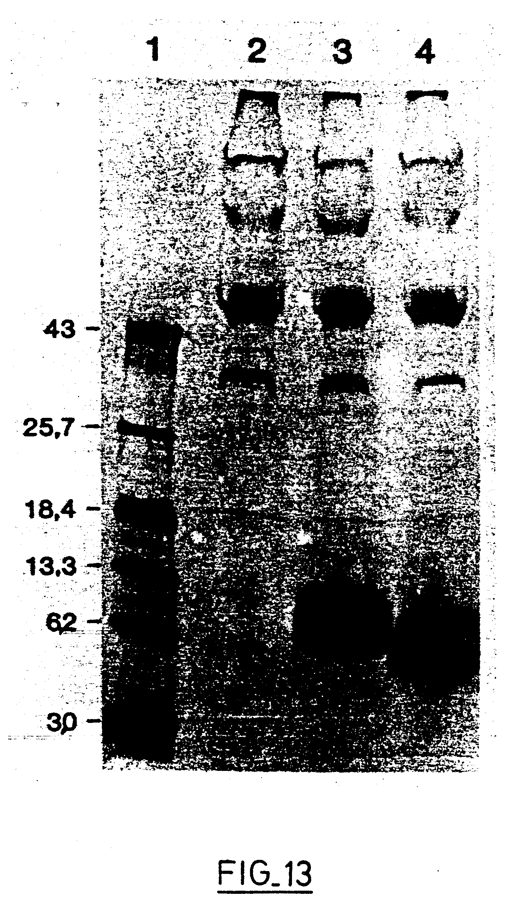

Les électrophorèses présentées dans les figures 12 et 13 ont été réalisées comme suit :

Pour la figure 12, les extraits ont été préparés de la façon suivante : 10 ml de milieu minimum (Yeast Nitrogen Base Difco sans acide aminé (6,7 g/l) glucose (10 g/l) supplémenté en casamino-acides 0,5 %, ont été ensemencés avec différentes souches et cultivés pendant 20 heures (phase stationnaire). Les cellules ont été centrifugées, le surnageant dialysé contre de l'eau (minimum de rétention : PM 1 000) puis séché par centrifugation sous vide. Les échantillons sont alors repris avec 50 »l de tampon de charge, dont 20 sont traités comme décrit précédemment et déposés sur gel d'acrylamide-SDS (15 % d'acrylamide, 0,1 % SDS) (22).

Les souches utilisées sont :
. puits 2 : TGY1sp4 transformé par un plasmide ne contenant pas la séquence de l'hirudine (contrôle);
. puits 3 : TGY1sp4 transformé par pTG886;
. puits 4 : TGY1sp4 transformé par pTG897;
. puits 1 : dans le puits 1 on a déposé les marqueurs de référence (LMW Kit de Pharmacia; de haut en bas : 94 000, 67 000, 43 000, 30 000, 20 100, 14 000).

Les bandes sont révélées par la coloration au bleu de Coomassie R-250.

Pour la figure 13, les extraits ont été préparés comme suit : 100 ml de milieu minimum + histidine 40 »g/ml ont été ensemencés avec différentes souches pour des cultures de nuit. Lorsque la densité cellulaire atteint environ 5.10⁶ (phase exponentielle de croissance) 40 »l de cystéine ³⁵S (9,8 mCi/ml : 1015 Ci/mmol.) sont ajoutés à chaque culture. Après 10 minutes, les cellules sont centrifugées, reprises dans 10 ml de milieu complet (30°C) et incubées à 30°C sous agitation. Après 3 heures, les 10 ml de surnageant sont dialysés contre de l'eau et concentrés jusqu'à un volume de 0,5 ml comme décrit pour la figure 12. Environ 35 000 cpm (40 »l) sont déposés sur gel d'acrylamide-SDS (15 % d'acrylamide, 0,1 % SDS). Les bandes de protéines sont visualisées après fluorographie.

Les souches utilisées sont :
. puits 2 : TGY1sp4 transformé par un plasmide ne contenant pas la séquence de l'hirudine;
. puits 3 : TGY1sp4 transformé par pTG886;
. puits 4 : TGY1sp4 transformé par pTG897;
. puits 1 : dans le puits 1 on a déposé des marqueurs dont les PM sont indiqués (x10³).

Cependant, quand les surnageants contenant ces polypeptides ont été testés pour leur activité anti-thrombine, aucune activité n'a pu être détectée.

Dans le cas du polypeptide excrété par TGY1sp4 pTG886, on s'attend à ce qu'il subisse les étapes normales de clivage du précurseur de la phéromone α, ce qui donnerait une molécule d'hirudine allongée de 8 amino-acides à son extrémité NH₂. Il n'est donc pas étonnant que le polypeptide sécrété par les cellules TGY1sp4 pTG886 ne soit pas actif.

Par contre, dans le cas du polypeptide sécrété par TGY1sp4/pTG897, on s'attend à ce que le polypeptide soit identique à la protéine naturelle et donc actif. Plusieurs hypothèses peuvent rendre compte de l'absence d'activité :
1°) La maturation de la protéine n'est pas totale, il peut rester des résidus Glu-Ala en NH₂ comme cela a été décrit pour l'EGF (16).
2°) La protéine n'est pas active car elle n'a pas la bonne conformation ou bien il y a dans le surnageant de culture des protéines ou des molécules de PM 1 000 qui inhibent l'activité.
3°) La protéine n'est pas complète parce qu'elle a subi une protéolyse intracellulaire et/ou extracellulaire.

### Exemple 4 - Activation par coupure au bromure de cyanogène

Si la cause de l'absence d'activité est liée à la présence des amino-acides supplémentaires en NH₂-terminal, il devrait être possible de retrouver de l'activité en soumettant le peptide sécrété par TGY1sp4/pTG886 au clivage par le bromure de cyanogène. En effet, ce réactif est spécifique des résidus méthionine et la protéine fusionnée codée par pTG886 ne contient qu'une seule méthionine. Cette réaction a été effectuée de la manière suivante : des levures contenant soit le plasmide pTG886, soit un plasmide de contrôle ne comprenant pas d'insert hirudine, sont mises en culture dans 10 ml de milieu pendant 24 heures. A ce moment, la culture atteint une densité de 7 à 10.10⁷ cellules.ml⁻¹. Le surnageant est séparé des cellules, dialysé intensivement contre de l'eau distillée puis lyophilisé.La poudre sèche est dissoute dans 1 ml d'acide formique à 70 % et une aliquote est utilisée pour la détermination du contenu en protéines totales (par la méthode de coloration au bleu de Coomassie, avec les réactifs vendus par Biorad) ; le reste de la préparation est traité avec 1 ml de solution fraîche de bromure de cyanogène à 30 mg/ml dans l'acide formique, 70 %.

Après élimination de l'oxygène par un flux d'azote, les tubes sont incubés dans l'obscurité pendant 4 heures, à température ambiante. Toutes les manipulations en présence de bromure de cyanogène sont effectuées avec des précautions appropriées et dans une hotte ventilée. La réaction de clivage par le bromure de cyanogène est arrêtée par l'addition de 10 volumes d'eau distillée puis la solution est lyophilisée.

Les peptides clivés sont redissous dans 10 ml d'eau distillée, et relyophilisés deux fois. Pour terminer, les peptides sont dissous dans un petit volume d'eau distillée et une aliquote est utilisée pour mesurer l'activité antithrombine. Le reste de l'échantillon est lyophilisé et soumis aux étapes de renaturation décrites plus bas.

Comme l'activité de l'hirudine dépend de la présence de ponts disulfures dans la molécule (1), il semblait probable que le peptide clivé au bromure de cyanogène devrait être renaturé correctement pour présenter une activité biologique. On a donc soumis les peptides clivés à une dénaturation en GuHCl 5 M suivie d'une renaturation, selon un procédé connu de l'homme de l'art.

En résumé, les peptides lyophilisés sont dissous dans 400 »l d'hydrochlorure de guanidinium (GuHCl) 5 M en Tris HCl 250 mM, pH 9,0 ; ensuite on rend la solution 2 mM en glutathion réduit et 0,2 mM en glutathion oxydé, dans un volume final de 2,0 ml (la concentration finale est 1,0 M GuHCl et 50 mM Tris).

Après 16 heures d'incubation à 23°C dans l'obscurité, les échantillons sont dialysés pendant 24 heures contre 3 fois 2 l de Tris HCl 50 mM, pH 7,5, NaCl 50 mM, à 23°C, et le dialysat final est clarifié par centrifugation.

On mesure ensuite l'activité antithrombine des surnageants. Le résultat de cette expérience, présenté dans le tableau I, montre clairement une récupération de l'activité antithrombine dans les surnageants des cellules infectées par le plasmide pTG886 alors qu'il n'y a pas d'activité avec le plasmide contrôle.

**TABLEAU I**

| ACTIVITE ANTITHROMBINE DES SURNAGEANTS DE CULTURES DE LEVURES | | | |
|---|---|---|---|
| Plasmide | Traitement du surnageant | Activité U/ml | Activité spécifique U/mg de protéines initiales |
| pTG886 | a)après clivage avant renaturation | < 0,3 | - |
| | b)après clivage et renaturation | 2,46 | 102,5 |
| contrôle | a)après clivage avant renaturation | < 0,3 | - |
| | b)après clivage et renaturation | < 0,15 | < 5,6 |

En conclusion, des cellules de levures portant un plasmide recombinant peuvent sécréter un peptide ayant l'activité biologique de l'hirudine, après une réaction de clivage et renaturation. Ceci indique que la présence d'amino-acides supplémentaires en NH₂-terminal suffirait à expliquer l'absence d'activité des polypeptides présents dans les cultures de TGY1sp4/pTG886 et peut-être aussi dans le cas des cultures de TGY1sp4/pTG897 (queues Glu-Ala).

### Exemple 5 - Introduction d'un nouveau site de coupure juste avant le premier amino-acide de la séquence codante de l'hirudine HV-2 - plasmide pTG1805

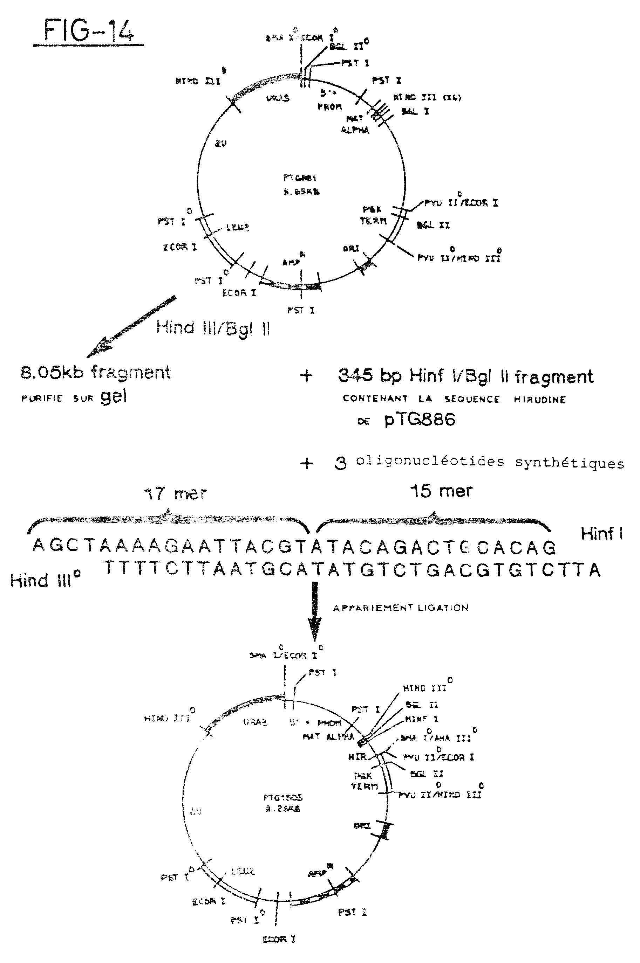

Dans la construction pTG897, il y a un risque que le peptide sécrété garde des queues Glu-Ala en NH₂, ce qui expliquerait l'absence d'activité antithrombine de ce matériel. Si cette hypothèse correspond à la réalité, il devrait être possible de récupérer de l'activité directement dans le surnageant en créant un site de coupure entre les résidus Glu-Ala et le premier amino-acide de l'hirudine HV-2 (Isoleucine). Ceci a été réalisé en ajoutant une séquence codant pour le doublet LysArg, qui est le site de reconnaissance de l'endopeptidase impliquée dans la maturation du précurseur de la phéromone (figure 2). La construction a été obtenue exactement comme décrit pour le plasmide pTG897, sauf en ce qui concerne la séquence de 2 oligonucléotides de synthèse (figure 14). Le plasmide résultant est appelé pTG1805. Le plasmide a servi à transformer la souche TGY1sp4 en ura⁺. Le matériel sécrété dans le surnageant a été analysé sur gel d'électrophorèse comme décrit précédemment et comparé au matériel obtenu avec TGY1sp4/pTG897 (figure 15).

Les souches utilisées sont :
. puits 1 : Marqueurs identiques à ceux de la figure 12;
. puits 2 : TGY1sp4 transformé par pTG897;
. puits 3 : TGY1sp4 transformé par pTG1805;
. puits 4 : contrôle ne produisant pas d'hirudine.

Les polypeptides spécifique de la souche TGY1sp4/pTG1805 migrent plus lentement que ceux spécifiques de la souche TGY1sp4/pTG897. Ce résultat suggère que le nouveau site de clivage n'est pas utilisé efficacement par l'endopeptidase correspondant. Néanmoins, le dosage de l'hirudine dans le surnageant par activité biologique révèle qu'une faible partie de ce matériel est actif, contrairement à ce qui est obtenu avec les cultures de TGY1sp4/pTG897, clairement inactif (tableau II).

**TABLEAU II**

| ACTIVITE ANTITHROMBINE DES SURNAGEANTS DE CULTURES DE LEVURES (10 ml) | | |
|---|---|---|
| Plasmide | Activité spécifique U/mg | Activité totale U (10 ml) |
| pTG856 | non détectable | - |
| pTG897 | non détectable | - |
| pTG1805 | 21 | 2,0 |

### Exemple 6 - Nouvelle construction impliquant un nouveau site de coupure plus efficace permettant la libération de l'hirudine mature

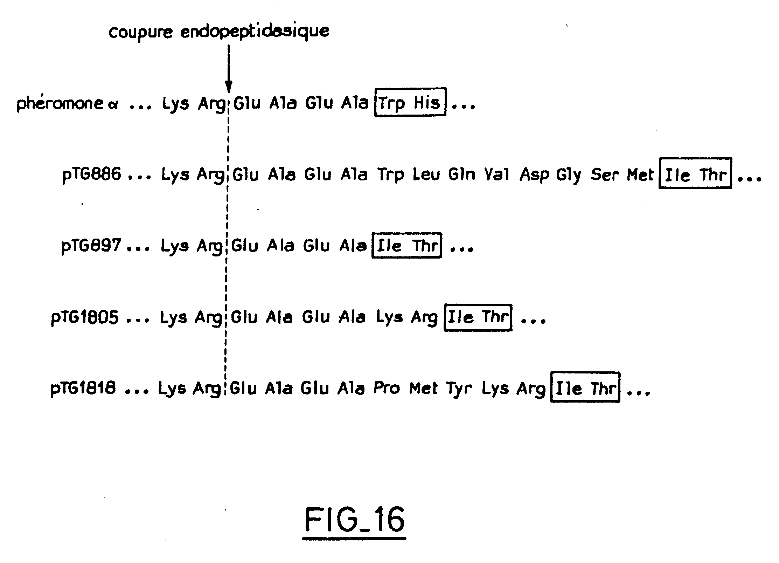

Dans la construction précédente (pTG897), on n'a obtenu que peu d'activité hirudine dans le surnageant, vraisemblablement parce que le deuxième site de clivage destiné à libérer de l'hirudine mature est reconnu avec peu d'efficacité. On a donc réalisé une nouvelle construction destinée à rendre ce site additionnel de clivage plus susceptible à l'endopeptidase en lui ajoutant, en amont, une séquence des 3 acides aminés Ser Leu Asp qui est naturellement présente en amont du premier doublet LysArg (figures 2 et 16).

La technique utilisée est la même que celle décrite précédemment sauf en ce qui concerne les oligonucléotides dont les séquences sont reportées ci-après : 
26-mère                    15-mère
5'-AGCTTCTTTGGATAAAAGAATTACGTATACAGACTGCACAG
AGAAACCTATTTTCTTAATGCATATGTCTGACGTGTCTTA
40-mère
La séquence en amino-acides dans la région du clivage est reportée figure 13. Le plasmide correspondant est appelé pTG1818 et ne diffère de pTG1805 que par l'insertion des nucléotides 5'-TTG GAT AAA correspondant aux codons Ser-Leu-Asp.

L'activité dosée dans les surnageants des cultures TGY1sp4 pTG1818 suivant les conditions standards déjà décrites est d'environ 200 unités pour 10 ml de culture, soit environ 100 fois supérieure à celle trouvée dans l'exemple précédent. Il est à noter que le dosage peut se faire avec 10 »l de milieu de culture non concentré.

### Exemple 7 - Construction aboutissant à la synthèse d'un précurseur dépourvu de séquences Glu-Ala-Glu-Ala

Dans les deux exemples précédents, on a montré que l'addition d'un nouveau site Lys Arg juste en amont du début de la séquence mature de l'hirudine permettait de libérer du matériel actif dans le surnageant. Cependant, dans ces exemples, si la coupure du précurseur se fait au premier site Lys Arg (distal par rapport au début de la séquence mature), on peut obtenir dans le milieu de culture un contaminant plus lourd, inactif, correspondant à des chaînes d'hirudine allongées à l'extrémité NH₂. Ceci est particulièrement clair dans le cas des cultures TGY1sp4 pTG1805 où le contaminant inactif est majoritaire. Cela reste également plausible dans le cas des cultures TGY1sp4 pTG1818 où le contaminant inactif n'est pas majoritaire mais pourraît être présent, comme décrit par d'autres dans le cas de l'EGF (15). Pour éviter cette perte de rendement que représente la synthèse de matériel inactif, on a décidé de procéder à une nouvelle construction, celle-ci aboutissant à la synthèse d'un précurseur qui ne diffère de celui synthétisé par les cellules TGY1sp4 pTG897 que par l'absence de la séquence Glu Ala Glu Ala, entre le site de coupure Lys Arg et le premier amino-acide de la séquence mature de l'hirudine.

Les souches suivantes ont été déposées à la Collection Nationale de Cultures de Microorganismes (CNCM) de l'INSTITUT PASTEUR, 28 rue du Docteur-Roux, PARIS 15ème, le 30 avril 1985 :
. TGY1sp4 pTG1818 : Saccharomyces cerevisiae, souche TGY1sp4 (Matα ura3-251-373-328-his 3-11-15) transformée en ura⁺ par un plasmide pTG1818 ; Dépôt n° I 441.
. TGY1sp4 pTG886 : Saccharomyces cerevisiae, souche TGY1sp4 (Matα ura3-251-373-328-his 3-11-15) transformée en ura⁺ par un plasmide pTG886 ; Dépôt n° I 442.

### REFERENCES

1. BAGDY D., BARABAS E., GRAF L., PETERSEN T.E. et MAGNUSSON S. (1976) Methods in Enzymology part B, vol. 45, pp. 669-678.
2. MARKWARDT F. (1970) Methods in Enzymology, ed. Perlman G.E. et Lorand L., Academic Press., vol. 19, pp. 924-932.
3. MARKWARDT F., HAUPTMANN J., NOWAK G., KLESSEN Ch. et WALSMANN P. (1982) Thromb. Hemostasis (Stuttgart) 47, 226-229.
4. WALSMANN P. et MARKWARDT F. (1981) Die Pharmazie 10, 653-660.
5. KLOSS Th. et MITTMANN U. (1982) Longenbecks Arch. Chirurg. 358, 548.
6. ISHIKAWA A., HAFTER R., SEEMULLER U., GOKEL J.M. et GRAEFF M. (1980) Thrombosis Research 19, 351-358.
7. MARKWARDT F., NOWAK G., STURZEBECKER J., GREISBADI U., WALSMANN P. et VOGEL G. (1984) Thromb. Hemostasis (Stuttgart) 52, 160-163.
8. NOWAK C. et MARKWARDT F. (1980) Expt. Path. 18, 438-443.
9. MARKWARDT F., NOWAK G., STURZENBECKER J., GREISSBACK U., WALSMANN P. et VOGEL G. (1984) Thromb. Hemostasis 52, 160-163.
10. SUTOR A.H., KNOP S. et ADLER D. (1981) Kontrolle Antithrombotica, 23rd Symp. Blutgerinnung, Hamburg, pp. 117-123.
11. PETERSEN T.E., ROBERTS H.R., SOTTRUP-JENSEN L., MAGNUSSON S. et BADGY D. (1976) Protides Biol. Fluids, Proc. Colloq. vol. 23, pp. 145-149.
12. CHANG J-Y. (1983) Febs Lett. 164, 307-313.
13. BASKOVA I.P., CHERKESOVA D.U. et MOSOLOV V.V. (1983) Thromb. Res. 30, 459-467.
14. BUSSEY H., SAVILLE D., GREENE D. et al. (1983) Mol. Cell. Biol. 3, 1362-1370.
15. BRAKE A.J., MERRYWEATHER J.P., COIT D.G., HEBERLEIN U.A., MARIARZ F.R., MULLENBACH G.T., URDEA M.S., VALENZUELA P. et BARR P.J. (1984) Proc. Natl. Acad. Sci. USA 81, 4642-4646.
16. BITTER G.A., CHEN K.K., BANKS A.R. et LAI P.H. (1984) Proc. Natl. Acad. Sci. USA 81, 5330-5334.
17. KURJAN J. et HERSKOWITZ I. (1982) Cell 30, 933-943.
18. BEGGS J. (1981) Genetic Engineering 2, 175-203.
19. BACH M.L., LACROUTE F. et BOTSTEIN D. (1979) Proc. Natl. Acad. Sci. (USA) 76, 386-390.
20. HITZEMAN R.A., HAGIE E.F., HAYFFLICK J.S. et al. (1982) Nucleic Acids Res. 10, 7791-7808.
21. ITO H., FUKUDA Y., MURATA K. et KIMURA A. (1983) J. Bacteriol. 153, 163-168.
22. LAEMMLI V.K.C. (1970) Nature 227, 680-685.

## Revendications (Revendications pour l'(les) Etat(s) contractant(s) suivant(s): BE, CH, DE, FR, GB, IT, LI, LU, NL, SE)

1. Levure transformée par un bloc fonctionnel d'ADN, intégré dans un plasmide comportant une origine de réplication dans la levure, ou intégré dans un chromosome de ladite levure, caractérisée en ce que ledit bloc comporte au moins :
- une séquence d'ADN codant pour l'hirudine, l'un de ses variants ou un précurseur de ceux-ci (gène H),
- une séquence leader (Lₑₓ) comportant les éléments nécessaires pour obtenir l'excrétion du produit du gène,
- une séquence d'ADN (Sₜᵣ) comportant les signaux assurant la transcription du gène H par la levure.

2. Levure transformée selon la revendication 1, caractérisée en ce que ledit bloc fonctionnel comporte au moins les séquences suivantes, depuis l'extrémité 5' jusqu'à l'extrémité 3' :
- une séquence (Sₜᵣ) comportant les signaux assurant la transcription du gène H par la levure,
- une séquence leader (Lₑₓ) comportant les éléments nécessaires pour obtenir l'excrétion du produit du gène,
- le gène codant pour l'hirudine, l'un de ses variants ou un précurseur de ceux-ci,
- le gène codant pour l'hirudine ou l'un de ses variants ou précurseurs de ceux-ci précédé d'un site de clivage S_{cl}.

3. Levure selon l'une des revendications 1 et 2, caractérisée en ce que la séquence Lₑₓ est celle de la phéromone sexuelle alpha de levure.

4. Levure selon l'une des revendications 1 à 3, caractérisée en ce que la séquence leader comporte le fragment pré de la phéromone sexuelle alpha de levure.

5. Levure selon l'une des revendications 1 à 4, caractérisée en ce que le bloc comporte la séquence pré-pro de la phéromone sexuelle alpha de levure à l'extrémité 5' du gène H.

6. Levure selon l'une des revendications 1 à 5, caractérisée en ce que le gène H est suivi d'une séquence de terminateur de levure.

7. Levure selon la revendication 6, caractérisée en ce que la séquence de terminateur de levure est celle du gène PGK.

8. Levure selon l'une des revendications 1 à 7, caractérisée en ce que le site de clivage est le dipeptide Lys-Arg.

9. Levure selon l'une des revendications 1 à 7, caractérisée en ce que le bloc fonctionnel est intégré dans un plasmide comportant au moins une origine de réplication dans les levures.

10. Levure selon la revendication 9, caractérisée en ce que l'origine de réplication est celle du plasmide 2».

11. Levure selon l'une des revendications 9 et 10, caractérisée en ce que le plasmide comporte, en outre, un caractère de sélection.

12. Levure selon la revendication 11, caractérisée en ce que le caractère de sélection est donné par le gène URA3.

13. Levure selon l'une des revendications 1 à 12, caractérisée en ce qu'il s'agit d'une levure du genre Saccharomyces.

14. Levure selon la revendication 13, caractérisée en ce qu'elle présente le type sexuel Matalpha.

15. Levure selon l'une des revendications 12 à 14, caractérisée en ce qu'il s'agit d'une souche de S. cerevisiae.

16. Procédé de préparation d'hirudine par fermentation d'une levure selon l'une des revendications 1 à 15 sur un milieu de culture et récupération de l'hirudine produite dans le milieu de culture.

17. Procédé de préparation d'hirudine par fermentation d'une levure, caractérisé en ce que :
a) on cultive sur un milieu de culture une souche de levure comportant un bloc fonctionnel d'ADN porté par un plasmide ou intégré dans un chromosome de ladite levure, caractérisé en ce qu'il comporte au moins la séquence :
---Sₜᵣ---Lₑₓ---S_{cl}---gène H---ter
. Sₜᵣ est une séquence d'ADN comportant les signaux assurant la transcription du gène H par la levure,
. Lₑₓ est une séquence leader permettant d'obtenir l'excrétion de l'hirudine mature ou sous forme d'un précurseur de l'hirudine maturable in vitro,
. S_{cl} est un signal d'ADN codant pour un site de clivage choisi parmi ATG et les séquences codant pour Lys-Arg ;
b) on récupère l'hirudine produite dans le milieu de culture sous forme mature ou sous forme d'un précurseur de l'hirudine maturable in vitro.

18. Procédé selon la revendication 17, caractérisé en ce que la séquence S_{cl}-gène H comporte une séquence codant pour la séquence Lys-Arg-Ile-Thr.

19. Circuit sanguin extracorporel, caractérisé en ce qu'au moins une partie du circuit en contact avec le sang présente une surface neutre d'hirudine obtenue par un procédé selon l'une des revendications 15 à 18.

20. Composition anticoagulante, caractérisée en ce qu'elle comporte des levures selon l'une des revendications 1 à 15 à titre d'agent librant de l'hirudine.

21. Composition pour la visualisation de la formation d'un caillot sanguin, caractérisée en ce qu'elle est constituée par de l'hirudine marquée obtenue par un procédé selon l'une des revendications 15 à 18.

## Revendications (Revendications pour l'(les) Etat(s) contractant(s) suivant(s): AT)

1. Procédé de préparation d'une levure transformée, caractérisée en ce que ladite levure est transformée par un bloc fonctionnel d'ADN, intégré dans un plasmide comportant une origine de réplication dans la levure, ou intégré dans un chromosome de ladite levure, ledit bloc comportant au moins :
- une séquence d'ADN codant pour l'hirudine, l'un de ses variants ou un précurseur de ceux-ci (gène H),
- une séquence leader (Lₑₓ) comportant les éléments nécessaires pour obtenir l'excrétion du produit du gène,
- une séquence d'ADN (Sₜᵣ) comportant les signaux assurant la transcription du gène H par la levure.

2. Procédé selon la revendication 1, caractérisée en ce que ledit bloc fonctionnel comporte au moins les séquences suivantes, depuis l'extrémité 5' jusqu'à l'extrémité 3' :
- une séquence (Sₜᵣ) comportant les signaux assurant la transcription du gène H par la levure,
- une séquence leader (Lₑₓ) comportant les éléments nécessaires pour obtenir l'excrétion du produit du gène,
- le gène codant pour l'hirudine, l'un de ses variants ou un précurseur de ceux-ci,
- le gène codant pour l'hirudine ou l'un de ses variants ou précurseurs de ceux-ci précédé d'un site de clivage S_{cl}.

3. Procédé selon l'une des revendications 1 et 2, caractérisé en ce que la séquence Lₑₓ est celle de la phéromone sexuelle alpha de levure.

4. Procédé selon l'une des revendications 1 à 3, caractérisé en ce que la séquence leader comporte le fragment pré de la phéromone sexuelle alpha de levure.

5. Procédé selon l'une des revendications 1 à 4, caractérisé en ce que le bloc comporte la séquence pré-pro de la phéromone sexuelle alpha de levure à l'extrémité 5' du gène H.

6. Procédé selon l'une des revendications 1 à 5, caractérisé en ce que le gène H est suivi d'une séquence de terminateur de levure.

7. Procédé selon la revendication 6, caractérisé en ce que la séquence de terminateur de levure est celle du gène PGK.

8. Procédé selon l'une des revendications 1 à 7, caractérisé en ce que le site de clivage est le dipeptide Lys-Arg.

9. Procédé selon l'une des revendications 1 à 7, caractérisé en ce que le bloc fonctionnel est intégré dans un plasmide comportant au moins une origine de réplication dans les levures.

10. Procédé selon la revendication 9, caractérisé en ce que l'origine de réplication est celle du plasmide 2».

11. Procédé selon l'une des revendications 9 et 10, caractérisé en ce que le plasmide comporte, en outre, un caractère de sélection.

12. Procédé selon la revendication 11, caractérisé en ce que le caractère de sélection est donné par le gène URA3.

13. Procédé selon l'une des revendications 1 à 12, caractérisé en ce qu'il s'agit d'une levure du genre Saccharomyces.

14. Procédé selon la revendications 13, caractérisé en ce qu'il présente le type sexuel Matalpha.

15. Procédé selon l'une des revendications 12 à 14, caractérisé en ce qu'il s'agit d'une souche de S. cerevisiae.

16. Procédé de préparation d'hirudine par fermentation d'une levure selon l'une des revendications 1 à 15 sur un milieu de culture et récupération de l'hirudine produite dans le milieu de culture.

17. Procédé de préparation d'hirudine par fermentation d'une levure, caractérisé en ce que :
a) on cultive sur un milieu de culture une souche de levure comportant un bloc fonctionnel d'ADN porté par un plasmide ou intégré dans un chromosome de ladite levure, caractérisé en ce qu'il comporte au moins la séquence :
---Sₜᵣ---Lₑₓ---S_{cl}---gène H---ter
. Sₜᵣ est une séquence d'ADN comportant les signaux assurant la transcription du gène H par la levure
. Lₑₓ est une séquence leader permettant d'obtenir l'excrétion de l'hirudine mature ou sous forme d'un précurseur de l'hirudine maturable in vitro,
. S_{cl} est un signal d'ADN codant pour un site de clivage choisi parmi ATG et les séquences codant pour Lys-Arg ;
b) on récupère l'hirudine produite dans le milieu de culture sous forme mature ou sous forme d'un précurseur de l'hirudine maturable in vitro.

18. Procédé selon la revendication 17, caractérisé en ce que la séquence S_{cl}-gène H comporte une séquence codant pour la séquence Lys-Arg-Ile-Thr.

19. Utilisation de l'hirudine obtenue par le procédé des revendications 15 à 18 pour préparer un circuit sanguin extracorporel, caractérisé en ce qu'au moins une partie du circuit en contact avec le sang présente une surface neutre de ladite hirudine.

20. Utilisation d'une levure selon l'une des revendications 1 à 15 à titre d'agent libérant de l'hirudine pour la préparation d'une composition anticoagulante.

21. Utilisation de l'hirudine marquée obtenue par un procédé selon l'une des revendications 15 à 18 pour la préparation d'une composition pour la visualisation de la formation d'un caillot sanguin.

## Claims (Claims for the following Contracting State(s): BE, CH, DE, FR, GB, IT, LI, LU, NL, SE)

1. Yeast transformed by a functional DNA block integrated in a plasmid containing an origin of replication in yeast, or integrated in a chromosome of the said yeast, characterized in that the said block contains at least:
- a DNA sequence coding for hirudin, one of its variants or a precursor of these (H gene),
- a leader sequence (Lₑₓ) containing the elements required for obtaining excretion of the gene product,
- a DNA sequence (Sₜᵣ) containing the signals which provide for transcription of the H gene by yeast.

2. Transformed yeast according to Claim 1, characterized in that the said functional block contains at least the following sequences, from the 5' end to the 3' end:
- a sequence (Sₜᵣ) containing the signals which provide for transcription of the H gene by yeast,
- a leader sequence (Lₑₓ) containing the elements required for obtaining excretion of the gene product,
- the gene coding for hirudin, one of its variants or a precursor of these,
- the gene coding for hirudin or one of its variants or precursors of these, preceded by a cleavage site S_{cl}.

3. Yeast according to either of Claims 1 and 2, characterized in that the sequence Lₑₓ is that of the alpha sex pheromone of yeast.

4. Yeast according to one of Claims 1 to 3, characterized in that the leader sequence contains the pre fragment of the alpha sex pheromone of yeast.

5. Yeast according to one of Claims 1 to 4, characterized in that the block contains the prepro sequence of the alpha sex pheromone of yeast at the 5' end of the H gene.

6. Yeast according to one of Claims 1 to 5, characterized in that the H gene is followed by a yeast terminator sequence.

7. Yeast according to Claim 6, characterized in that the yeast terminator sequence is that of the PGK gene.

8. Yeast according to one of Claims 1 to 7, characterized in that the cleavage site is the dipeptide Lys-Arg.

9. Yeast according to one of Claims 1 to 7, characterized in that the functional block is integrated in a plasmid containing at least one origin of replication in yeasts.

10. Yeast according to Claim 9, characterized in that the origin of replication is that of the 2» plasmid.

11. Yeast according to one of Claims 9 and 10, characterized in that the plasmid contains, in addition, a selectable character.

12. Yeast according to Claim 11, characterized in that the selectable character is given by the URA3 gene.

13. Yeast according to one of Claims 1 to 12, characterized in that it is a yeast of the genus Saccharomyces.

14. Yeast according to Claim 13, characterized in that it possesses the Matalpha mating type.

15. Yeast according to one of Claims 12 to 14, characterized in that it is a strain of S. cerevisiae.

16. Process for preparing hirudin by fermentation of a yeast according to one of Claims 1 to 15 in a culture medium and recovery of the hirudin produced in the culture medium.

17. Process for preparing hirudin by fermentation of a yeast, characterized in that:
a) a yeast strain containing a functional DNA block carried by a plasmid or integrated in a chromosome of the said yeast is cultured in a culture medium, characterized in that the DNA block contains at least the sequence:
---Sₜᵣ---Lₑₓ---S_{cl}---H gene---ter
· Sₜᵣ is a DNA sequence containing the signals which provide for transcription of the H gene by yeast,
· Lₑₓ is a leader sequence enabling the excretion of hirudin, mature or in the form of a hirudin precursor which can be matured in vitro, to be obtained,
· S_{cl} is a DNA signal coding for a cleavage site chosen from ATG and the sequences coding for Lys-Arg;
b) the hirudin produced is recovered in the culture medium in mature form or in the form of a hirudin precursor which can be matured in vitro.

18. Process according to Claim 17, characterized in that the sequence S_{cl}-H gene contains a sequence coding for the sequence Lys-Arg-Ile-Thr.

19. Extracorporeal blood circuit, characterized in that at least part of the circuit in contact with the blood possesses a neutral surface of hirudin obtained by a process according to one of Claims 16 to 18.

20. Anticoagulant composition, characterized in that it contains yeasts according to one of Claims 1 to 15 as an agent which liberates hirudin.

21. Composition for visualizing the formation of a blood clot, characterized in that it consists of labelled hirudin obtained by a process according to one of Claims 16 to 18.

## Claims (Claims for the following Contracting State(s): AT)

1. Process for preparing a transformed yeast, characterized in that the said yeast is transformed by a functional DNA block integrated in a plasmid containing an origin of replication in yeast, or integrated in a chromosome of the said yeast, the said block containing at least:
- a DNA sequence coding for hirudin, one of its variants or a precursor of these (H gene),
- a leader sequence (Lₑₓ) containing the elements required for obtaining excretion of the gene product,
- a DNA sequence (Sₜᵣ) containing the signals which provide for transcription of the H gene by yeast.

2. Process according to Claim 1, characterized in that the said functional block contains at least the following sequences, from the 5' end to the 3' end:
- a sequence (Sₜᵣ) containing the signals which provide for transcription of the H gene by yeast,
- a leader sequence (Lₑₓ) containing the elements required for obtaining excretion of the gene product,
- the gene coding for hirudin, one of its variants or a precursor of these,
- the gene coding for hirudin or one of its variants or precursors of these, preceded by a cleavage site S_{cl}.

3. Process according to either of Claims 1 and 2, characterized in that the sequence Lₑₓ is that of the alpha sex pheromone of yeast.

4. Process according to one of Claims 1 to 3, characterized in that the leader sequence contains the pre fragment of the alpha sex pheromone of yeast.

5. Process according to one of Claims 1 to 4, characterized in that the block contains the prepro sequence of the alpha sex pheromone of yeast at the 5' end of the H gene.

6. Process according to one of Claims 1 to 5, characterized in that the H gene is followed by a yeast terminator sequence.

7. Process according to Claim 6, characterized in that the yeast terminator sequence is that of the PGK gene.

8. Process according to one of Claims 1 to 7, characterized in that the cleavage site is the dipeptide Lys-Arg.

9. Process according to one of Claims 1 to 7, characterized in that the functional block is integrated in a plasmid containing at least one origin of replication in yeasts.

10. Process according to Claim 9, characterized in that the origin of replication is that of the 2» plasmid.

11. Process according to one of Claims 9 and 10, characterized in that the plasmid contains, in addition, a selectable character.

12. Process according to Claim 11, characterized in that the selectable character is given by the URA3 gene.

13. Process according to one of Claims 1 to 12, characterized in that the yeast in question is of the genus Saccharomyces.

14. Process according to Claim 13, characterized in that the yeast possesses the Matalpha mating type.

15. Process according to one of Claims 12 to 14, characterized in that the yeast in question is a strain of S. cerevisiae.

16. Process for preparing hirudin by fermentation of a yeast according to one of Claims 1 to 15 in a culture medium and recovery of the hirudin produced in the culture medium.

17. Process for preparing hirudin by fermentation of a yeast, characterized in that:
a) a yeast strain containing a functional DNA block carried by a plasmid or integrated in a chromosome of the said yeast is cultured in a culture medium, characterized in that the DNA block contains at least the sequence:
---Sₜᵣ---Lₑₓ---S_{cl}---H gene---ter
· Sₜᵣ is a DNA sequence containing the signals which provide for transcription of the H gene by yeast,
· Lₑₓ is a leader sequence enabling the excretion of hirudin, mature or in the form of a hirudin precursor which can be matured in vitro, to be obtained,
· S_{cl} is a DNA signal coding for a cleavage site chosen from ATG and the sequences coding for Lys-Arg;
b) the hirudin produced is recovered in the culture medium in mature form or in the form of a hirudin precursor which can be matured in vitro.

18. Process according to Claim 17, characterized in that the sequence S_{cl}-H gene contains a sequence coding for the sequence Lys-Arg-Ile-Thr.

19. Use of hirudin obtained by the process of Claims 15 to 18 for preparing an extracorporeal blood circuit, characterized in that at least part of the circuit in contact with the blood possesses a neutral surface of the said hirudin.

20. Use of a yeast according to one of Claims 1 to 15 as an agent which liberates hirudin for the preparation of an anticoagulant composition.

21. Use of labelled hirudin obtained by a process according to one of Claims 15 to 18 for the preparation of a composition for visualizing the formation of a blood clot.

## Patentansprüche (Patentansprüche für folgende(n) Vertragsstaat(en): BE, CH, DE, FR, GB, IT, LI, LU, NL, SE)

1. Hefe, die transformiert worden ist durch einen funktionellen DNA-Block, der integriert ist in ein Plasmid, das einen Replikationsursprung in der Hefe aufweist, oder integriert ist in ein Chromosom der genannten Hefe, dadurch gekennzeichnet, daß der genannte Block umfaßt mindestens:
- eine DNA-Sequenz, die für Hirudin, eine seiner Varianten oder einen Vorläufer desselben (Gen H) codiert,
- eine Leader-Sequenz (Lₑₓ), welche die für die Erzielung der Exkretion des Produkts des Gens erforderlichen Elemente aufweist, und
- eine DNA-Sequenz (Sₜᵣ), welche die Signale aufweist, welche die Transkription des Gens H durch die Hefe gewährleisten.

2. Transformierte Hefe nach Anspruch 1, dadurch gekennzeichnet, daß der genannte funktionelle Block ab dem 5'-Ende bis zu dem 3'-Ende mindestens die folgenden Sequenzen aufweist:
- eine Sequenz (Sₜᵣ), welche die Signale aufweist, welche die Transkription des Gens H durch die Hefe gewährleisten,
- eine Leader-Sequenz (Lₑₓ), welche die für die Erzielung der Exkretion des Produkts des Gens erforderlichen Elemente aufweist,
- das Gen, das für Hirudin, eine seiner Varianten oder einen Vorläufer desselben codiert, und
- das Gen, das für Hirudin oder eine seiner Varianten oder Vorläufer desselben codiert, dem eine Schnittstelle S_{cl} vorausgeht.

3. Hefe nach einem der Ansprüche 1 und 2, dadurch gekennzeichnet, daß die Sequenz Lₑₓ diejenige des α-Sexual-Pheromons von Hefe ist.

4. Hefe nach einem der Ansprüche 1 bis 3, dadurch gekennzeichnet, daß die Leader-Sequenz das Fragment ("pre" des α-Sexual-Pheromons von Hefe umfaßt.

5. Hefe nach einem der Ansprüche 1 bis 4, dadurch gekennzeichnet, daß der Block die Sequenz pre-pro des α-Sexual-Pheromons von Hefe an dem 5'-Ende des Gens H aufweist.

6. Hefe nach einem der Ansprüche 1 bis 5, dadurch gekennzeichnet, daß auf das Gen H eine Terminator-Sequenz von Hefe folgt.

7. Hefe nach Anspruch 6, dadurch gekennzeichnet, daß die Terminator-Sequenz von Hefe diejenige des Gens PGK ist.

8. Hefe nach einem der Ansprüche 1 bis 7, dadurch gekennzeichnet, daß die Schnittstelle (Spaltungsstelle) das Dipeptid Lys-Arg ist.

9. Hefe nach einem der Ansprüche 1 bis 7, dadurch gekennzeichnet, daß der funktionelle Block in ein Plasmid integriert ist, das mindestens einen Replikationsursprung in den Hefen aufweist.

10. Hefe nach Anspruch 9, dadurch gekennzeichnet, daß der Replikationsursprung derjenige des Plasmids 2 » ist.

11. Hefe nach einem der Ansprüche 9 und 10, dadurch gekennzeichnet, daß das Plasmid außerdem einen Selektionscharakter aufweist.

12. Hefe nach Anspruch 11, dadurch gekennzeichnet, daß der Selektionscharakter durch das Gen URA3 gegeben ist.

13. Hefe nach einem der Ansprüche 1 bis 12, dadurch gekennzeichnet, daß es sich dabei um eine Hefe des Genus Saccharomyces handelt.

14. Hefe nach Anspruch 13, dadurch gekennzeichnet, daß sie den Sexual-Typ Matalpha darstellt.

15. Hefe nach einem der Ansprüche 12 bis 14, dadurch gekennzeichnet, daß es sich dabei um einen Stamm von S. cerevisiae handelt.

16. Verfahren zur Herstellung von Hirudin durch Fermentation einer Hefe nach einem der Ansprüche 1 bis 15 auf einem Kulturmedium und Gewinnung (Abtrennung) des in dem Kulturmedium gebildeten Hirudins.

17. Verfahren zur Herstellung von Hirudin durch Fermentation einer Hefe, dadurch gekennzeichnet, daß man:
a) einen Hefe-Stamm, der einen funktionellen DNA-Block aufweist, der von einem Plasmid getragen wird oder integriert ist in ein Chromosom der genannten Hefe, auf einem Kulturmedium kultiviert, wobei die Hefe dadurch charakterisiert ist, daß sie mindestens die Sequenz aufweist:
---Sₜᵣ---Lₑₓ---S_{cl}---Gen H---ter in der:
. Sₜᵣ steht für eine DNA-Sequenz, welche die Signale aufweist, welche die Transkription des Gens H durch die Hefe gewährleisten,
. Lₑₓ steht für eine Leader-Sequenz, welche die Erzielung der Exkretion von reifem Hirudin oder in Form eines in vitro reifungsfähigen Vorläufers des Hirudins erlaubt,
. S_{cl} steht für ein DNA-Signal, das für eine Schnittstelle codiert, die ausgewählt wird aus ATG und den Sequenzen, die für Lys-Arg codieren; und
b) das in dem Kulturmedium gebildete Hirudin in reifer Form oder in Form eines in vitro reifungsfähigen Hirudinvorläufers gewinnt (abtrennt).

18. Verfahren nach Anspruch 17, dadurch gekennzeichnet, daß die Sequenz S_{cl}-Gen H eine Sequenz aufweist, die für die Sequenz Lys-Arg-Ile-Thr codiert.

19. Extrakorporaler Blutkreislauf, dadurch gekennzeichnet, daß mindestens ein Teil des Kreislaufes, der mit dem Blut in Kontakt steht, eine neutrale Hirudin-Oberfläche aufweist, die durch ein Verfahren nach einem der Ansprüche 15 bis 18 erhalten worden ist.

20. Antikoagulans-Zusammensetzung, dadurch gekennzeichnet, daß sie Hefen nach einem der Ansprüche 1 bis 15 als Mittel enthält, das Hirudin freisetzt.

21. Zusammensetzung zur Sichtbarmachung der Bildung eines Blutgerinnsels, dadurch gekennzeichnet, daß sie aus dem markierten Hirudin besteht, das nach einem Verfahren nach einem der Ansprüche 15 bis 18 erhalten worden ist.

## Patentansprüche (Patentansprüche für folgende(n) Vertragsstaat(en): AT)

1. Verfahren zur Herstellung einer transformierten Hefe, dadurch gekennzeichnet, daß die genanne Hefe transformiert wird durch einen funktionellen DNA-Block, der integriert ist in ein Plasmid, das einen Replikationsursprung in der Hefe aufweist, oder integriert ist in ein Chromosom der genannten Hefe, wobei der genannte Block umfaßt mindestens:
- eine DNA-Sequenz, die für Hirudin, eine seiner Varianten oder einen Vorläufer desselben (Gen H) codiert,
- eine Leader-Sequenz (Lₑₓ), welche die für die Erzielung der Exkretion des Produkts des Gens erforderlichen Elemente aufweist, und
- eine DNA-Sequenz (Sₜᵣ), welche die Signale aufweist, welche die Transkription des Gens H durch die Hefe gewährleisten.

2. Verfahren nach Anspruch 1, dadurch gekennzeichnet, daß der genannte funktionelle Block ab dem 5'-Ende bis zu dem 3'-Ende mindestens die folgenden Sequenzen aufweist:
- eine Sequenz (Sₜᵣ), welche die Signale aufweist, welche die Transkription des Gens H durch die Hefe gewährleisten,
- eine Leader-Sequenz (Lₑₓ), welche die für die Erzielung der Exkretion des Produkts des Gens erforderlichen Elemente aufweist,
- das Gen, das für Hirudin, eine seiner Varianten oder einen Vorläufer desselben codiert, und
- das Gen, das für Hirudin oder eine seiner Varianten oder Vorläufer desselben codiert, dem eine Schnittstelle S_{cl} vorausgeht.

3. Verfahren nach einem der Ansprüche 1 und 2, dadurch gekennzeichnet, daß die Sequenz Lₑₓ diejenige des α-Sexual-Pheromons von Hefe ist.

4. Verfahren nach einem der Ansprüche 1 bis 3, dadurch gekennzeichnet, daß die Leader-Sequenz das Fragment "pre" des α-Sexual-Pheromons von Hefe umfaßt.

5. Verfahren nach einem der Ansprüche 1 bis 4, dadurch gekennzeichnet, daß der Block die Sequenz pre-pro des α-Sexual-Pheromons von Hefe an dem 5'-Ende des Gens H aufweist.

6. Verfahren nach einem der Ansprüche 1 bis 5, dadurch gekennzeichnet, daß auf das Gen H eine Terminator-Sequenz von Hefe folgt.

7. Verfahren nach Anspruch 6, dadurch gekennzeichnet, daß die Terminator-Sequenz von Hefe diejenige des Gens PGK ist.

8. Verfahren nach einem der Ansprüche 1 bis 7, dadurch gekennzeichnet, daß die Schnittstelle (Spaltungsstelle) das Dipeptid Lys-Arg ist.

9. Verfahren nach einem der Ansprüche 1 bis 7, dadurch gekennzeichnet, daß der funktionelle Block in ein Plasmid integriert wird, das mindestens einen Replikationsursprung in den Hefen aufweist.

10. Verfahren nach Anspruch 9, dadurch gekennzeichnet, daß der Replikationsursprung derjenige des Plasmids 2 » ist.

11. Verfahren nach einem der Ansprüche 9 und 10, dadurch gekennzeichnet, daß das Plasmid außerdem einen Selektionscharakter aufweist.

12. Verfahren nach Anspruch 11, dadurch gekennzeichnet, daß der Selektionscharakter durch das Gen URA3 gegeben ist.

13. Verfahren nach einem der Ansprüche 1 bis 12, dadurch gekennzeichnet, daß es sich dabei um eine Hefe des Genus Saccharomyces handelt.

14. Verfahren nach Anspruch 13, dadurch gekennzeichnet, daß sie den Sexual-Typ Matalpha darstellt.

15. Verfahren nach einem der Ansprüche 12 bis 14, dadurch gekennzeichnet, daß es sich dabei um einen Stamm von S. cerevisiae handelt.

16. Verfahren zur Herstellung von Hirudin durch Fermentation einer Hefe nach einem der Ansprüche 1 bis 15 auf einem Kulturmedium und Gewinnung (Abtrennung) des in dem Kulturmedium gebildeten Hirudins.

17. Verfahren zur Herstellung von Hirudin durch Fermentation einer Hefe, dadurch gekennzeichnet, daß man
a) einen Hefe-Stamm, der einen funktionellen DNA-Block aufweist, der von einem Plasmid getragen wird oder integriert ist in ein Chromosom der genannten Hefe, auf einem Kulturmedium kultiviert, wobei die Hefe dadurch charakterisiert ist, daß sie mindestens die Sequenz aufweist:
---Sₜᵣ---Lₑₓ---S_{cl}---Gen H---ter in der:
. Sₜᵣ steht für eine DNA-Sequenz, welche die Signale aufweist, welche die Transkription des Gens H durch die Hefe gewährleisten,
. Lₑₓ steht für eine Leader-Sequenz, welche die Erzielung der Exkretion von reifem Hirudin oder in Form eines in vitro reifungsfähigen Vorläufers des Hirudins erlaubt, und
. S_{cl} steht für ein DNA-Signal, das für eine Schnittstelle codiert, die ausgewählt wird aus ATG und den Sequenzen, die für Lys-Arg codieren; und
b) das in dem Kulturmedium gebildete Hirudin in reifer Form oder in Form eines in vitro reifungsfähigen Hirudinvorläufers gewinnt (abtrennt).

18. Verfahren nach Anspruch 17, dadurch gekennzeichnet, daß die Sequenz S_{cl}-Gen H eine Sequenz aufweist, die für die Sequenz Lys-Arg-Ile-Thr codiert.

19. Verwendung des nach dem Verfahren der Ansprüche 15 bis 18 erhaltenen Hirudins zur Herstellung eines extrakorporalen Blutkreislaufes, dadurch gekennzeichnet, daß mindestens ein Teil des Kreislaufes, der mit dem Blut in Kontakt steht, eine neutrale Hirudin-Oberfläche aufweist.

20. Verwendung einer Hefe nach einem der Ansprüche 1 bis 15 als Mittel, das Hirudin freisetzt, zur Herstellung einer Antikoagulans-Zusammensetzung.

21. Verwendung des nach einem Verfahren nach einem der Ansprüche 15 bis 18 erhaltenen markierten Hirudins zur Herstellung einer Zusammensetzung für die Sichtbarmachung der Bildung eines Blutgerinnsels.
